(19) Europäisches Patentamt
European Patent Office
Office européen des brevets

(11) Publication number: **0 289 227 B1**

# (12) EUROPEAN PATENT SPECIFICATION

(45) Date of publication of patent specification: **08.04.92**

(51) Int. Cl.[5]: **C07D 233/64**, C07D 403/06, A61K 31/415, A61K 31/435

(21) Application number: **88303646.9**

(22) Date of filing: **22.04.88**

## (54) Substituted imidazolyl-alkyl-piperazine and -diazepine derivatives.

(30) Priority: **24.04.87 US 42181**

(43) Date of publication of application:
**02.11.88 Bulletin 88/44**

(45) Publication of the grant of the patent:
**08.04.92 Bulletin 92/15**

(84) Designated Contracting States:
**AT BE CH DE ES FR GB GR IT LI LU NL SE**

(56) References cited:
**BE-A- 820 242**
**US-A- 3 362 956**
**US-A- 3 491 098**

(73) Proprietor: **SYNTEX PHARMACEUTICALS LTD.**
**Syntex House St. Ives Road**
**Maidenhead Berkshire SL6 1RD(GB)**

(72) Inventor: **Pascal, Jean-Claude**
**23-25 Allee des Hautes Bruyeres**
**F-94230 Cachan(FR)**
Inventor: **Lee, Chi-Ho**
**3758 La Donna Street,**
**Palo Alto, CA 94306(US)**
Inventor: **Alps, Brian J.**
**2 St. Ninians Avenue,**
**Linlithgow, Scotland(GB)**
Inventor: **Pinhas, Henri**
**39, rue Truffaut**
**F-75017 Paris(FR)**
Inventor: **Whiting, Roger L.**
**1848 Fallenleaf Lane,**
**Los Altos, CA 94022(US)**
Inventor: **Beranger, Serge**
**12 Allee des Cedres**
**F-91120 Bretigny-sur-Orge(FR)**

(74) Representative: **Armitage, Ian Michael et al**
**MEWBURN ELLIS & CO. 2/3 Cursitor Street**
**London EC4A 1BO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to substituted imidazolyl-alkyl-piperazine and diazepine derivatives, the pharmaceutically acceptable salts thereof, methods of making these compounds, and pharmaceutical compositions containing these compounds. The compounds of this invention are calcium entry blockers having selectivity for cerebral blood vessels, and have protective activity against some of the deleterious effects resultant upon cerebral ischemia. The compounds of this invention are, therefore, useful for treating mammals having a variety of disease states, such as stroke, epilepsy or epileptic psychotic symptoms, hypertension, angina, migraine, arrhythmia, thrombosis, embolism, acute cardiac failure, irritable bowel syndrome, neurodegenerative diseases such as Alzheimer's or Huntingtons's chorea, diuresis, ischemia such as focal and global ischemia or cerebrovascular ischemia induced by cocaine abuse, and also for treatment of spinal injuries.

Substituted piperazines have been described as having a variety of pharmaceutical activities.

For example, U.S. Patent No. 3,362,956 and its CIP 3,491,098, disclose a series of substituted piperazines to be useful as tranquilizers, sedatives, adrenolytic agents, hypothermic agents, anti-convulsants, hypotensive agents and cardiovascular agents. For example, in the '956 patent, compounds of the formula

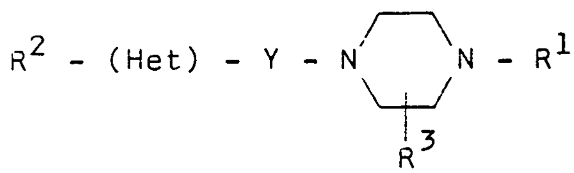

wherein $R^1$ is a lower-alkyl, hydroxy-lower alkyl, phenyl or substituted-phenyl, phenyl-lower-alkyl, or substituted-phenyl-lower-alkyl, benzhydryl or substituted benzhydryl, phenyl-lower-alkenyl or substituted-phenyl-lower-alkenyl, or pyridyl radical; $R^2$ is hydrogen or from one to two lower-alkyl radicals; Y is lower-alkylene of from one to six carbon atoms; and Het is a heterocyclic radical selected from the group consisting of bicyclic aromatic nitrogen heterocyclic radicals having fused five and six membered rings and containing from two to three ring nitrogen atoms which can be in any position of the two rings, for example, radicals derived from indazole (e.g. 2-azaindole, 4-azaindole, 5-azaindole, 6-azaindole, 7-azaindole), pyrrolo-[2,3-d]-pyrimidine, benzimidazole and pyrido[2,1-c]-s-triazole; a benz[g]-3-indolyl radical; a 4(5)-imidazolyl radical; a 3-thianaphthenyl radical; a 3-quinolyl radical; a 3,4-dihydro-1-isoquinolyl radical; or 1,2,3,4-tetrahydro-1-isoquinolyl radical or such heterocyclic radicals substituted in any available position by from one to three substituents, defined hereinafter as $R^2$, for example, methyl, ethyl, propyl, and isobutyl; lower-alkoxy, for example, methoxy, ethoxy, propoxy, and butoxy; halogen, including fluorine, chlorine, bromine, and iodine; lower-alkylmercapto, for example, methylmercapto, ethylmercapto, propylmercapto, and isobutylmercapto; lower-alkylsulfinyl, for example, methylsulfinyl, ethylsulfinyl, propylsulfinyl, and isobutylsulfinyl; lower-alkylsulfonyl, for example, methylsulfonyl, ethylsulfonyl, propylsulfonyl, isopropylsulfonyl; trifluoromethyl; hydroxy; methylenedioxy; or ethylenedioxy, wherein the lower-alkyl moiety of the said substituents contain from one to four carbon atoms; are disclosed.

In the '098 patent compounds of the formula

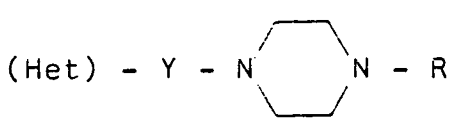

wherein R is hydrogen or a lower-alkyl, hydroxy-lower-alkyl, phenyl, phenyl-lower-alkyl, benzhydryl, phenyl-lower-alkenyl, cycloalkyl-lower-alkyl, or pyridyl radical; Y is lower-alkylene of from one to six carbon atoms; and Het is a 4(5)-imidazolyl radical; are disclosed.

None of the prior art teaches 4(5)-imdazolyl-substituted piperazine or diazepine derivatives substituted in the manner of this invention which are useful for treating mammals having a variety of disease states, such as stroke, epilepsy or epileptic psychotic symptoms, hypertension, angina, migraine, arrhythmia, thrombosis, embolism, acute cardiac failure, irritable bowel syndrome, neurodegenerative diseases such as

Alzheimer's or Huntington's chorea, diuresis, ischemia such as focal and global ischemia or cerebrovascular ischemia induced by cocaine abuse, and also for treatment of spinal injuries.

## SUMMARY OF THE INVENTION

A first aspect of this invention encompasses compounds having the structures represented by Formula A:

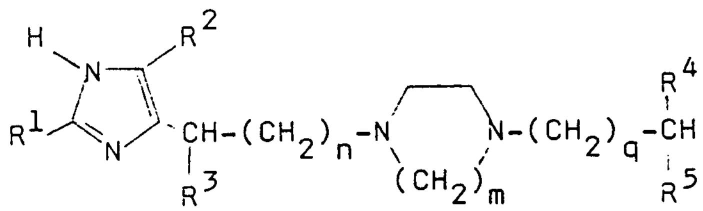

FORMULA A

wherein:

- $R^1$ is aryl;
- $R^2$ is aryl, lower alkyl, or hydrogen;
- $R^3$ is lower alkyl, hydroxy, or hydrogen;
- $R^4$ is aryl;
- $R^5$ is aryl;
- m is two or three;
- n is zero, one or two, provided that when $R^3$ is hydroxy, n is one or two; and
- q is zero, one, two, or three;

and the pharmaceutically acceptable salts thereof.

A further aspect of the present invention encompasses methods of making compounds of Formula A

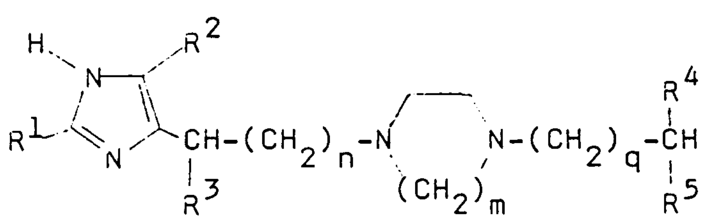

FORMULA A

wherein:

- $R^1$ is aryl;
- $R^2$ is aryl, lower alkyl, or hydrogen;
- $R^3$ is lower alkyl, hydroxy, or hydrogen;
- $R^4$ is aryl;
- $R^5$ is aryl;
- m is two or three;
- n is zero, one or two, provided that when $R^3$ is hydroxy, n is one or two; and
- q is zero, one, two, or three;

and the pharmaceutically acceptable salts thereof, which comprises

(a) condensing a compound of the formula

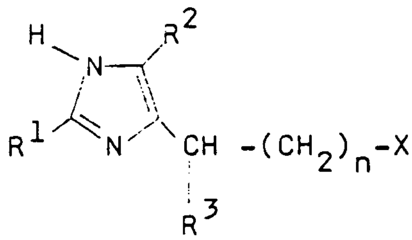

wherein n, $R^1$ and $R^2$ are as defined above, $R^3$ is hydrogen or lower alkyl, and X is a halogen atom, with a compound of the formula

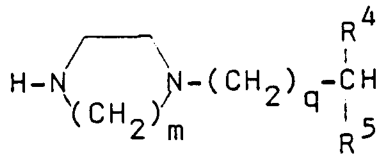

wherein m, q, $R^4$ and $R^5$ are as defined above; or

b) reducing a compound of the formula

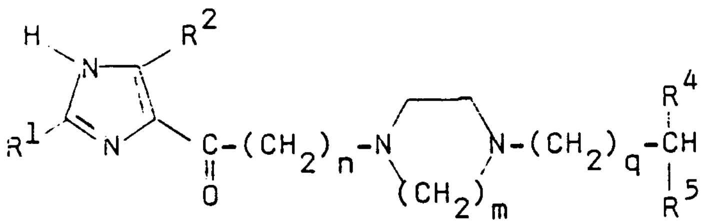

wherein $R^1$, $R^2$, $R^4$, $R^5$, m, and q are as defined above and n is one or two, to form a compound of formula A wherein $R^3$ is hydroxy; or

c) converting a free base compound of formula A to its acid addition salt; or

d) converting an acid addition salt of a compound of formula A to a free base compound of formula A; or

e) converting an acid addition salt of a compound of formula A to another acid solution salt of a compound of formula A.

A still further aspect of the present invention encompasses methods of treating a mammals having a variety of disease states, such as stroke, epilepsy or epileptic psychotic symptoms, hypertension, angina, migraine, arrhythmia, thrombosis, embolism, acute cardiac failure, irritable bowel syndrome, neurodegenerative diseases such as Alzheimer's or Huntington's chorea, diuresis, ischemia such as focal and global ischemia or cerebrovascular ischemia induced by cocaine abuse, and also for treatment of spinal injuries, comprising administering a therapeutically effective amount of compound of Formula A to a mammal.

Another aspect of the present invention encompasses pharmaceutical formulations comprising a compound of Formula A and a pharmaceutically acceptable excipient.

## DETAILED DESCRIPTION OF THE INVENTION

### Definitions

The numbering of the piperazines and diazepines of the present invention is as follows:

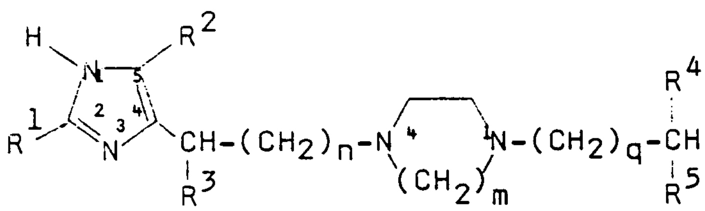

The compounds of the invention will be named using the above-shown numbering system as 1-[di-aryl]-alkyl-4-[(2-and/or 5-substituted- imidazolyl)-optionally-substituted-alkyl]-piperazines and -diazepines. Some representative compounds are named as follows:

the compound of Formula A where $R^1$ is 4-methylphenyl, $R^2$ is methyl, $R^3$ is hydrogen, $R^4$ is phenyl, $R^5$ is phenyl, m is 2, n is 0 and q is 0, is named "1-diphenylmethyl-4-[(2-(4-methylphenyl)-5-methyl-1H-imidazol-4-yl)methyl]piperazine";

the compound of Formula A where $R^1$ is 4-methylphenyl, $R^2$ is hydrogen, $R^3$ is hydrogen, $R^4$ is phenyl, $R^5$ is phenyl, m is 2, n is 0, and q is 0, is named "1-dihpenylmethyl-4-[(2-(4-methylphenyl)-1H-imidazol-4-yl)methyl]piperazine";

the compound of Formula A where $R^1$ is phenyl, $R^2$ is methyl, $R^3$ is methyl, $R^4$ is phenyl, $R^5$ is phenyl, m is 2, n is 0 and q is 0, is named "1-diphenylmethyl-4-[1-(2-phenyl-5-methyl-1H-imidazol-4-yl)ethyl]-piperazine";

the compound of Formula A where $R^1$ is phenyl, $R^2$ is methyl, $R^3$ is hydroxy, $R^4$ is phenyl, $R^5$ is phenyl, m is 2, n is 1 and q is 0, is named "1-diphenylmethyl-4-[2-(2-phenyl-5-methyl-1H-imidazol-4-yl)-2-hydroxyethyl]piperazine";

the compound of Formula A where $R^1$ is phenyl, $R^2$ is methyl, $R^3$ is hydrogen, $R^4$ is 4-fluorophenyl, $R^5$ is 4-fluorophenyl, m is 2, n is 0 and q is 3, is named "1-[4,4-di-(4-fluorophenyl)butyl]-4-[(2-phenyl-5-methyl-1H-imidazol-4-yl)methyl]piperazine"; and

the compound of Formula A where $R^1$ is 2,4-dihydroxyphenyl, $R^2$ is methyl, $R^3$ is hydrogen, $R^4$ is phenyl, $R^5$ is phenyl, m is 3, n is 0 and q is 0, is named "1-diphenylmethyl-4-[(2-(2,4-dihydroxyphenyl)-5-methyl-1H-imidazol-4-yl)methyl]diazepine".

Compounds of the invention where $R^3$ is lower alkyl or hydroxy, and/or where $R^4$ and $R^5$ are different will have one or two chiral centers and may display optical activity. The optical isomers may be separated using conventional methods. For purposes of the present invention, any compound having optical activity shall include each individual isomer as well as mixtures thereof.

As used herein, the term "alkyl" means a branched or unbranched saturated hydrocarbon radical having from 1 to 6 carbon atoms. Examples include methyl, ethyl, n-propyl, iso-propyl, n-butyl, t-butyl, n-pentyl, iso-pentyl, n-hexyl, and the like.

As used herein, the term "alkoxy" means the group -OR wherein R is alkyl as defined above. Examples include methoxy, ethoxy, n-propoxy, i-propoxy, n-butoxy, t-butoxy, n-pentyloxy, n-hexyloxy, and the like.

As used herein, the term "lower" modifies alkyl and alkoxy and refers to those radicals having four carbon atoms or less.

As used herein, the term "halo" means fluoro, chloro, bromo and/or iodo.

As used herein, the term "aryl" refers to phenyl and optionally mono-, di-, and tri-substituted phenyl, wherein the optional substituents are lower alkyl, lower alkoxy, hydroxy, trifluoromethyl, or halo. Examples include 3-chlorophenyl, 3-trifluoromethylphenyl, 4-methoxyphenyl, 4-chlorophenyl, 3,4-dimethoxyphenyl, 4-hydroxyphenyl, 4-methylphenyl, 3-t-butylphenyl, 4-hexylphenyl, and the like.

As used herein, the term "treatment" or "treating" means any treatment of a disease in a mammal, and includes:

(i) preventing the disease, i.e., causing the clinical symptoms of the disease not to develop;

(ii) inhibiting the disease, i.e., arresting the development of clinical symptoms; and/or

(iii) relieving the disease, i.e., causing the regression of clinical symptoms.

As used herein, the terms "pharmaceutically acceptable salts" refers to those salts that retain biological effectiveness and properties of the neutral parent compounds and which are not biologically or otherwise undesirable. Pharmaceutically acceptable acid addition salts may be formed with inorganic acids, such as hydrochloric acid, hydrobromic acid, sulfuric acid, nitric acid, phosphoric acid, and the like, and with organic acids such as acetic acid, propionic acid, glycolic acid, pyruvic acid, oxalic acid, malic acid, malonic acid, succinic acid, maleic acid, fumaric acid, tartaric acid, citric acid, lactic acid, benzoic acid, cinnamic acid, mandelic acid, methanesulfonic acid, ethanesulfonic acid, para-toluenesulfonic acid, salicylic acid, and the

like. The salts may be single or multiple salts of one or more anions, e.g., the mono-, di-, or tri- salts from the above-described acids.

Presently Preferred Embodiments

Presently preferred embodiments of this invention are compounds of Formula A wherein $R^1$ is aryl; further preferred are the compounds where $R^1$ is aryl, m is 2; q is 0; n is 0; $R^2$ is methyl; and $R^3$ is hydrogen. The pharmaceutically acceptable salts of these compounds are also preferred, especially the mono-, di- and tri-hydrochlorides, fumarates, and lactates.

Particularly preferred are those compounds where $R^1$ is 4-methylphenyl or phenyl and $R^4$ and $R^5$ are the same, e.g., both phenyl, i.e., 1-(diphenylmethyl)-4-[(2-(4-methylphenyl)-5-methyl-1H-imidazol-4-yl)-methyl]piperazine and 1-(diphenylmethyl)-4-[(2-phenyl-5-methyl-1H-imidazol-4-yl)methyl]piperazine and the trihydrochloride or trilactate salts thereof.

Other preferred compounds include those where $R^1$ is aryl; especially those where $R^3$ is lower alkyl, particularly methyl and isopropyl; m is 2; q is 0; n is 0; and $R^1$, $R^4$ and $R^5$ are all phenyl.

Still other preferred compounds include those where q is 3; n is 0; $R^1$ is phenyl; $R^3$ hydrogen; and $R^4$ and $R^5$ are both 4-fluorophenyl.

Another preferred compound is that wherein m is 3; q is 0; n is 0; $R^1$ is phenyl; $R^2$ is methyl; $R^3$ is hydrogen; and $R^4$ and $R^5$ are both phenyl.

Utility and Methods of Administration

General Utility

The compounds of this invention are useful for treating mammals having a variety of vascular disease states, and have protective activity against some of the deleterious effects resultant upon cerebral ischemia. Disease states that may be treated include stroke, migraine, epilepsy or epileptic psychotic symptoms, hypertension, angina, arrhythmia, thrombosis, embolism, acute cardiac failure, irritable bowel syndrome, neurodegenerative diseases such as Alzheimer's or Huntington'a chorea, diuresis, ischemia such as focal and global ischemia or cerebrovascular ischemia induced by cocaine abuse. The compounds of this invention are also useful for treating spinal injuries, and are particularly useful for treating cerebrovascular disease states, for example, stroke.

Generally, vascular disease states are found in mammals, including: domestic commercial animals such as horses, cattle, sheep and pigs; domestic house animals such as dogs, cats, and the like; and particularly humans.

Activity Testing

Activity testing for treating stroke, migraine, epilepsy or epileptic psychotic symptoms, hypertension, angina, arrhythmia, thrombosis, embolism, acute cardiac failure, irritable bowel syndrome, neurodegenerative diseases such as Alzheimer's or Huntington'a chorea, diuresis, ischemia such as focal and global ischemia, cerebrovascular ischemia induced by cocaine abuse, or spinal injuries can be undertaken in vitro and/or in vivo using assay procedures known in the literature. The following are examples of such assay procedures.

Activity for treating vascular disease states can be determined in vitro by determining selective vascular relaxant activity, and in vivo by determining general cardiovascular activity.

In vitro calcium antagonistic activity of the compounds of this invention is determined by an assay using rat aortic strip, which is a modification of that described by R. Kent, et al., Federation Proceedings, 40, 724 (1981). Cerebrovascular selectivity of action is determined by comparing potencies on rabbit basilar artery and rabbit ear artery using a modification of the procedure described by R. Towart, et al., Arzneim. Forsh., 32(I), 338-346 (1982).

In vivo protective effects of the compounds of this invention against the deleterious effects of cerebral ischemia are determined by use of the standard gerbil brain ischemia model. This assay is a modification of that described by T. Kirino, Brain Res., 239, 57-69P (1982).

General Administration

The compounds of this invention are administered at a therapeutically effective dosage, i.e., a dosage

sufficient to provide treatment for the disease states previously described. Administration of the active compounds and salts described herein can be via any of the accepted modes of administration for agents that serve similar utilities.

Generally, a daily dose is from 0.02 to 50 mg/kg of body weight per day of the active compound of Formula A. Most conditions respond to treatment comprising a dosage level on the order of 0.1 to 4 mg/kilogram of body weight per day. Thus, for administration to a 70 kg person, the dosage range would be about 1.4 to 3500 mg per day, preferably about 7.0 to 280 mg per day.

Depending on the specific disease state, administration can be via any accepted systemic route, for example, via parenteral, oral, intravenous, or nasal routes, in the form of solid, semi-solid or liquid dosage forms, such as for example, tablets, suppositories, pills, capsules, powders, solutions, suspensions, aerosols, emulsions or the like, preferably in unit dosage forms suitable for simple administration of precise dosages. The compositions will include a conventional pharmaceutical carrier or excipient and an active compound of Formula A and, in addition, may include other medicinal agents, pharmaceutical agents, carriers, adjuvants, etc.

If desired, the pharmaceutical composition to be administered may also contain minor amounts of non-toxic auxiliary substances such as wetting or emulsifying agents, pH buffering agents and the like, such as for example, sodium acetate, sorbitan monolaurate, triethanolamine oleate, etc.

The compounds of this invention are generally administered as a pharmaceutical composition which comprises a pharmaceutical excipient in combination with a compound of Formula A. The level of the drug in a formulation can vary within the full range employed by those skilled in the art, e.g., from about 0.01 weight percent (w%) to about 99.99 w% of the drug based on the total formulation and about 0.01 w% to 99.99 w% excipient.

## Oral Administration

The preferred manner of administration, for the conditions detailed above, is oral using a convenient daily dosage regimen which can be adjusted according to the degree of affliction. For such oral administration, a pharmaceutically acceptable, non-toxic composition is formed by the incorporation of the compound of formula A in any of the normally employed excipients, such as, for example, pharmaceutical grades of mannitol, lactose, starch, magnesium stearate, sodium saccarine, talcum, cellulose, glucose, gelatin, sucrose, magnesium carbonate, and the like. Such compositions take the form of solutions, suspensions, tablets, pills, capsules, powders, sustained release formulations and the like. Such compositions may contain between 0.01 wt/% and 99.99 wt/% of the compound of Formula A, but preferably such compositions will contain between 25 wt/% and about 80 wt/%.

Preferably the compositions will take the form of a pill or tablet and thus the composition will contain, along with the active ingredient, a diluent such as lactose, sucrose, dicalcium phosphate, and the like; a disintegrant such as starch or derivatives thereof; a lubricant such as magnesium stearate and the like; and a binder such as a starch, polyvinylpyrrolidone, gum acacia, gelatin, cellulose and derivatives thereof, and the like.

## Suppositories

For systemic administration via suppository, traditional binders and carriers include, for example, polyalkaline glycol or triglycerides [e.g., PEG 1000 (96%) and PEG 4000 (4%)]. Such suppositories may be formed from mixtures containing active ingredients in the range of from about 0.5 wt/% to about 10 wt/%; preferably from about 1 wt/% to about 2 wt/%.

## Liquids

Liquid pharmaceutically administerable compositions can, for example, be prepared by dissolving, dispersing, etc. an active compound (about 0.5% to about 20%), as described above, and optional pharmaceutical adjuvants in a carrier, such as, for example, water, saline, aqueous dextrose, glycerol, ethanol and the like, to thereby form a solution or suspension.

Actual methods of preparing such dosage forms are known, or will be apparent, to those skilled in this art; for example, see Remington's Pharmaceutical Sciences, Mack Publishing Company, Easton, Pennsylvania, 16th Ed., 1980. The composition to be administered will, in any event, contain a quantity of the active compound(s) in a pharmaceutically effective amount for relief of the particular condition being treated in accordance with the teachings of this invention.

Methods of Preparation

The compounds of this invention can be made as shown in Reaction Schemes I to VI, wherein $R^1$, $R^2$, $R^3$, $R^4$, and $R^5$ have the same meaning as set forth above in the Summary Of The Invention, and X is halo.

REACTION SCHEME I

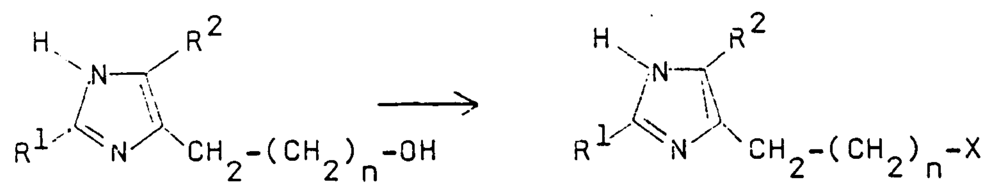

1 2

Step I-A

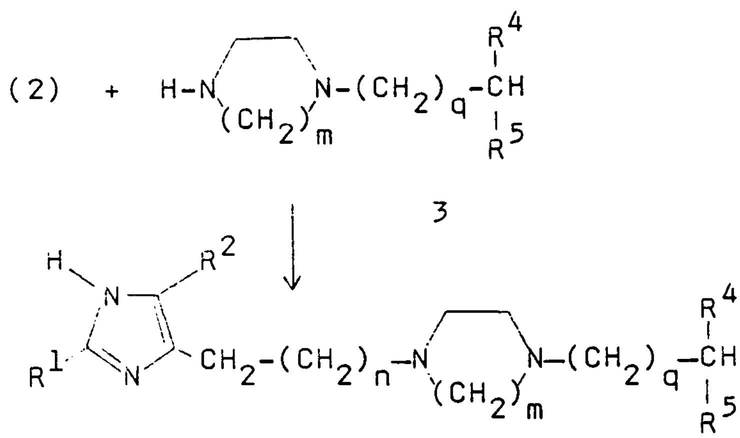

3

$A^1$

Step I-B

I. Preparation of Formula A Where $R^3$ is Hydrogen

Referring to Reaction Scheme I, in Step I-A a mixture of a compound of Formula 1 with an inert hydrocarbon solvent (for example, benzene, toluene, chloroform and the like) is refluxed with a slight excess of a halogenating agent, for example, a thionyl halide (such as thionyl chloride), to produce the corresponding alkyl halide derivative, compound of Formula 2. Compounds of Formula 1 are obtained by reacting the appropriate amidine with the appropriate dione using the procedures of Dziuron and Schunack [Arch. Pharm., 306, 347 (1973) and Arch. Pharm., 307, 46 (1973)] Imbach, et al Bull. Soc. Chim. France, 3 1059 (1971), Cornforth and Huang, J. Chem. Soc., (1948) 731-735, Ewins, J. Chem. Soc., 99 2052 (1911), of U.S. patent 4,107,307. The reaction is conducted at a temperature from about 0 °C to the reflux temperature of the solvent, but preferably between about 40 °C and 65 °C.

In Step I-B, the compound of Formula 2 is condensed with a compound of Formula 3, for example, at a temperature from about 25° to 80 °C, preferably at the reflux temperature of the solvent system used.

The 1-substituted piperazines of Formula 3 are commercially available or can be made by the procedures of Hamlin, et al., J. Am. Chem. Soc., 71, 2731 (1949) or Cheeseman, J. Chem Soc., (1975), 115-123. Diazepine analogs (i.e., those compounds where m is 3) can be made by this method using diazepine as the starting material instead of piperazine. An alkaline solution is made by dissolving a compound of

Formula 3 in a polar solvent (for example, methanol, ethanol, or a mixture such as ethanol and water, methanol and water, acetone in water, dimethylformamide in water, isopropanol in water, tetrahydrofuran in water; in the ratio of from about 10:90 to about 90:10, preferably about 60:40), and adding a base (such as sodium hydroxide, potassium hydroxide, sodium carbonate, sodium bicarbonate, potassium carbonate, potassium bicarbonate, and the like). The alkaline solution is heated to reflux.

A solution of the compound of Formula 2 dissolved in the same polar solvent is added dropwise to the refluxing solution of the compound of Formula 3. After about 1 to 24 hours, preferably about 2 to 5 hours, the condensed product of Formula $A^1$ is separated from the reaction mixture either by precipitation or evaporation under reduced pressure. The reaction mixture is allowed to stand at room temperature, for example, for about 8 to 24 hours, preferably overnight. If the product precipitated, the resulting crystals are removed by filtration and recrystallized in an alcohol, preferably methanol or ethanol giving the free base of Formula $A^1$. If the product is an oil, the oil is washed with water and dissolved in diethyl ether. Acid is added and the product is precipitated as the acid addition salt using ethanol.

The free base can be converted to the salt by dissolving the free base in a suitable organic solvent, such as ethanol or ether, and extracting with acidic aqueous solution. The use of heat may be required to dissolve the free base, depending upon the acid chosen.

The salt can be converted back to the free base by suspending it, for example in ether, and adding an excess of a dilute aqueous base, such as potassium carbonate, until the salt dissolves. The organic layer is separated, washed with water, dried and evaporated to yield the free base.

The salt of a compound of formula A can be converted to another salt by methods known in the art for interconversion of salts.

REACTION SCHEME II

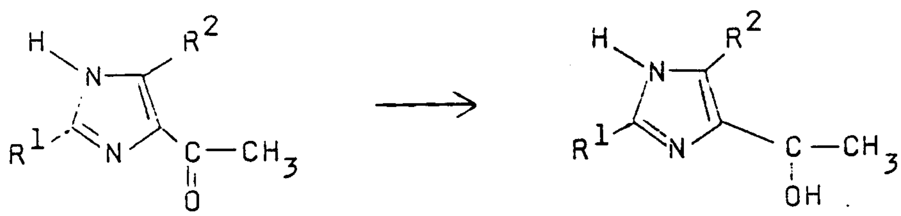

4 5

Step II-A

5

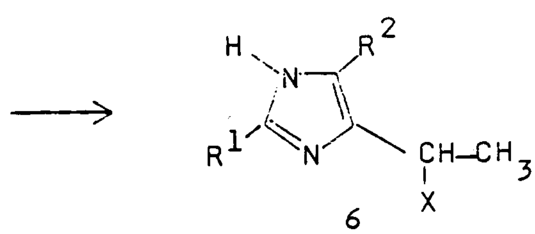

6

Step II-B

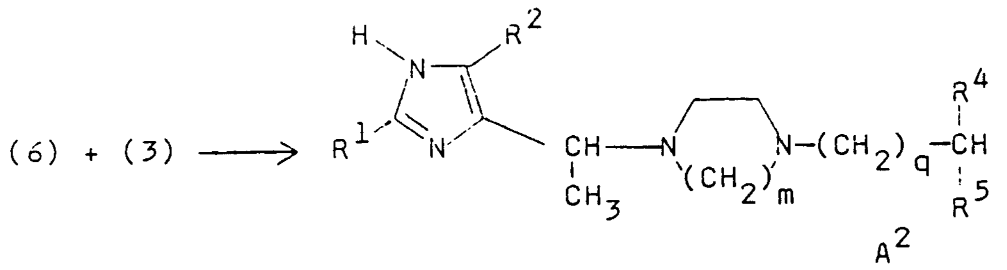

Step II-C

II. Preparation of Formula A Where $R^3$ is Methyl and n is 0

Referring to Reaction Scheme II, in Step II-A a compound of Formula 4 [obtained using the procedure of Vecchio, et al., Chim. Ind. (Milan), 58(6), 451 (1976) or of Haruki, et al., Nippon Kagaku Zasshiu, 86(9), 942-946 (1965) (Japan)] is reduced by contacting it with a reducing agent, such as a hydride (for example, potassium borohydride, or lithium aluminum hydride) in an alcoholic solution (for example, methanol, ethanol, isopropanol, and the like). The solution is stirred for about 2 to 16 hours, e.g., overnight, and the resulting solid material, of Formula 5, is removed by filtration (using reduced pressure if necessary), washed, dried and used in the next step without further purification.

In Step II-B, the crude compound of Formula 5 is dissolved in an inert solvent (for example, chloroform, dichloromethane, benzene, toluene, and the like). A halogenating agent, such as thionyl chloride, is added to the solution and the resulting mixture is heated at reflux for a period of time between 1 and 10 hours, preferably between about 4 and 6 hours. After cooling, the solvent is removed under reduced pressure and the residue is triturated in acetone to give a compound of Formula 6.

In Step II-C, the compound of Formula 6 and a compound of Formula 3 are reacted together under the conditions described in Step I-B to give a compound according to Formula $A^2$. An oil product may be separated, dissolved in ether, and acidified to precipitate. The reaction time is about 1 to 24 hours, preferably about 4 to 5 hours.

REACTION SCHEME III

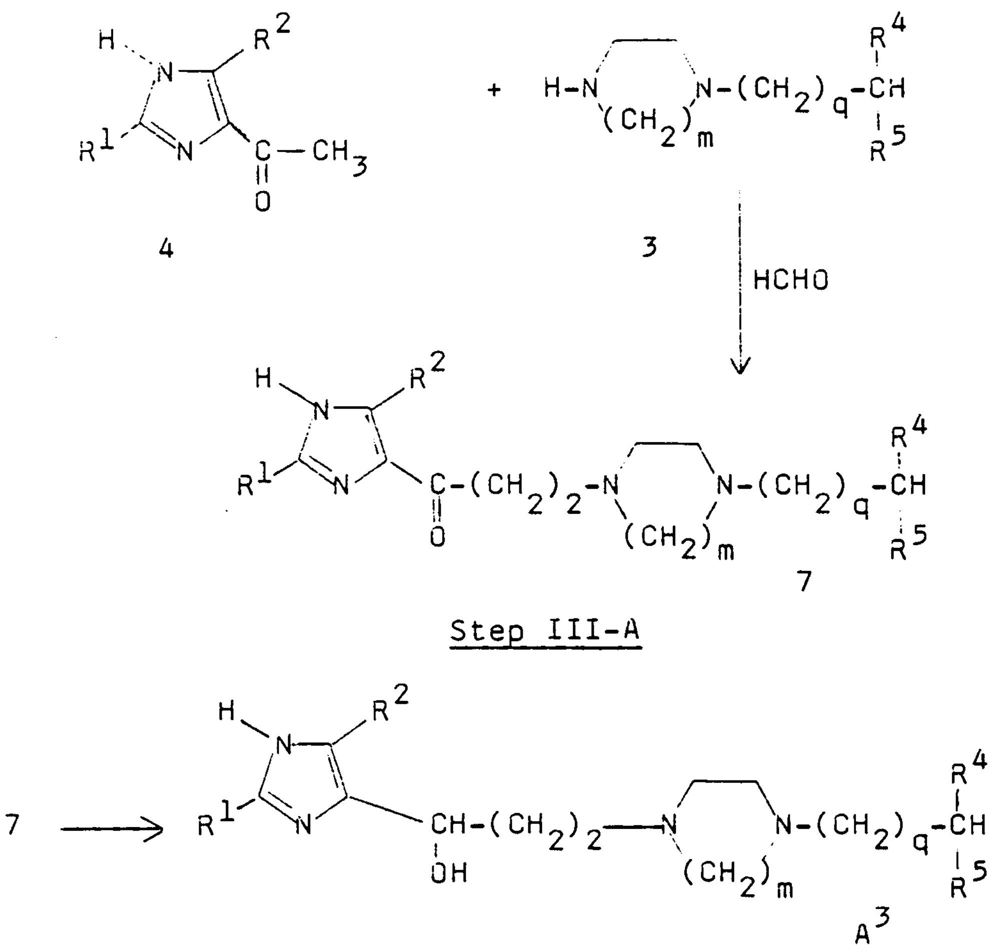


4 3 7

Step III-A

$A^3$

Step III-B

III. Preparation of Formula A Where $R^3$ is Hydroxyl and n is 2

Referring to Reaction Scheme III, in Step III-A a methyl ketone, such as a compound of Formula 4, an amine, such as a compound of Formula 3, and formaldehyde are reacted together under the conditions typically used for a Mannich reaction, to give a compound of Formula 7. Thus, the ketone of Formula 7 is dissolved in a solvent such as water, methanol, ethanol, or acetic acid and formaldehyde is introduced. The amine is then added and the reaction mixture is refluxed. If necessary a small amount of an acid such as hydrochloric acid, may be introduced to assure that the reaction mixture is not basic.

In Step III-B, the compound of Formula 7 is reduced under conditions similar to those described in Step II-A, to give a compound of Formula $A^3$.

REACTION SCHEME IV

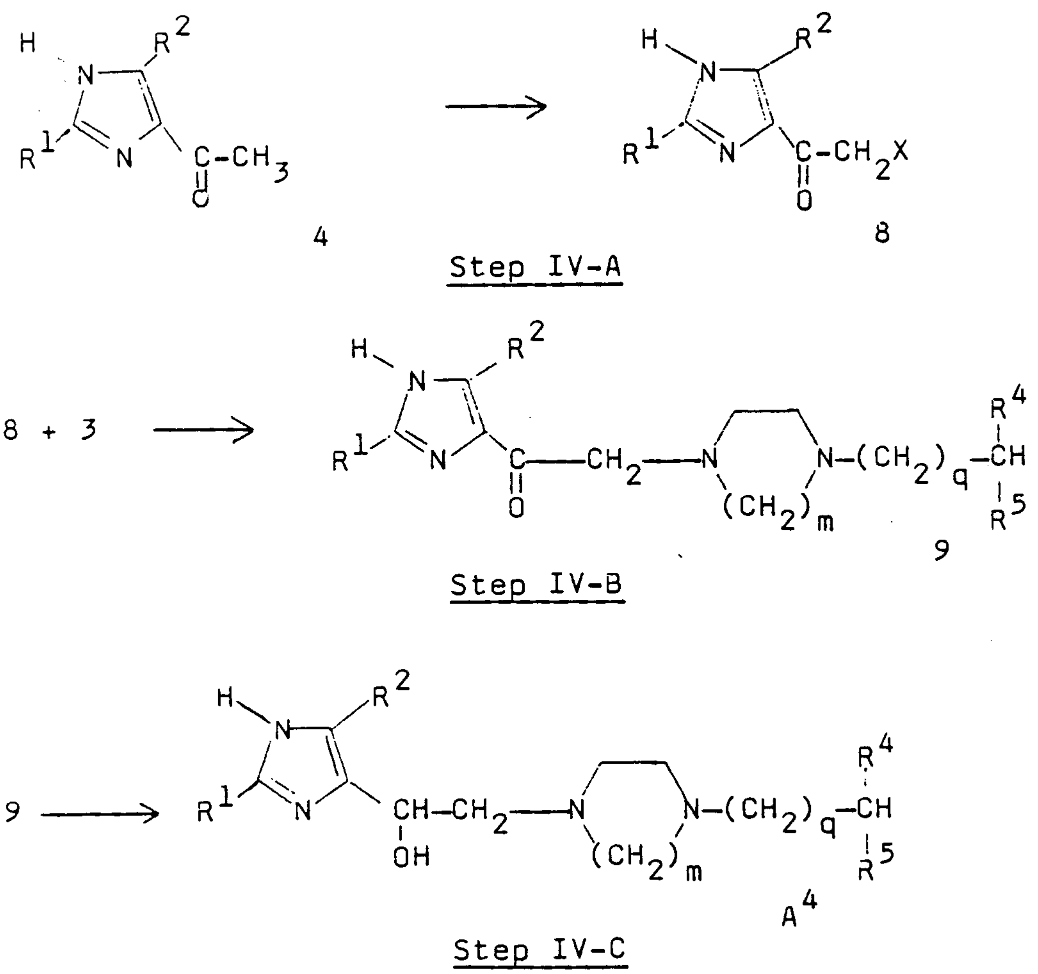

4

8

Step IV-A

8 + 3

9

Step IV-B

9

$A^4$

Step IV-C

IV. Preparation of Formula A Where $R^3$ is Hydroxyl and n is 1

Referring to Reaction Scheme IV, in Step IV-A a compound of Formula 4 is halogenated, by contacting it with a halogenating agent, for example, thionyl halide, under conditions similar to those described in Step I-A, to give a compound of the Formula 8.

In Step IV-B, a compound of Formula 8 and a compound of Formula 3 are condensed by contacting them under conditions similar to those described for Step I-B, to give a compound of Formula 9.

In Step IV-C, a compound of Formula 9 is reduced, by contacting it with a reducing agent under conditions similar to those described for Step II-A to give a compound of Formula $A^4$.

## REACTION SCHEME V

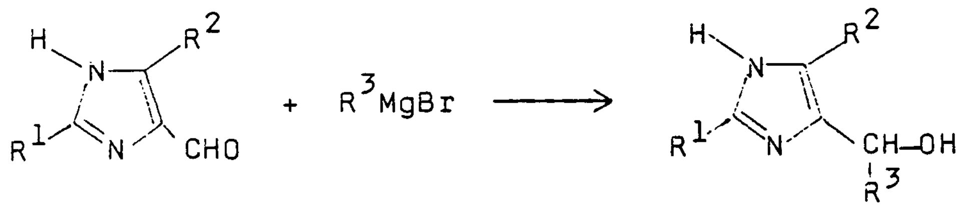

10 11 12

Step V-A

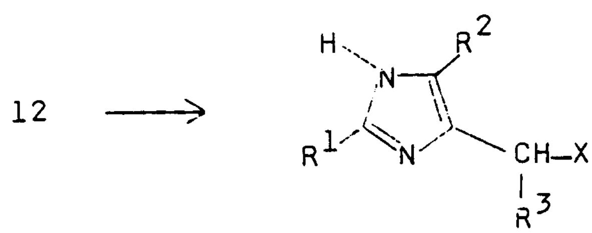

13

Step V-B

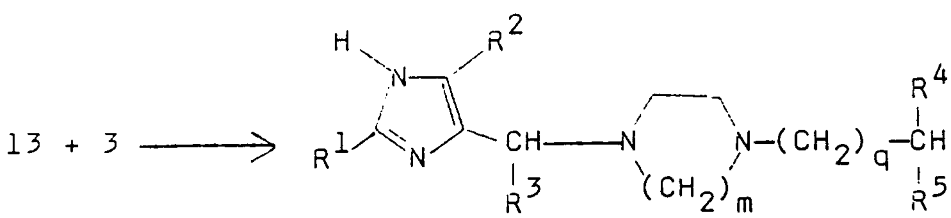

$A^5$

Step V-C

### V. Preparation of Formula A Where $R^3$ is Lower Alkyl

Referring to Reaction Scheme V, in Step V-A, the Grignard reagent of a lower alkyl halide, the compound of Formula 11, is contacted with an imidazole compound of Formula 10 [obtained using the procedure of Cornforth and Huang, J. Chem. Soc., (1948), 731-735] in a neutral solvent (for example, diethyl ether, tetrahydrofuran, tetrahydropyran, and the like) and refluxed for between 15 minutes and 2 hours, preferably between 20 minutes and 40 minutes, and is then cooled and poured into ice water. The aqueous layer is extracted with a suitable organic solvent, such as diethyl ether. When the solvent is removed under reduced pressure, a residue is formed that can be recrystallized in ethanol yielding a compound of Formula 12.

In Step V-B, a compound of Formula 12 is halogenated under conditions similar to those described for Step I-A, to give a compound of Formula 13.

In Step V-C, a compound of Formula 13 and a compound of Formula 3 are contacted under conditions similar to those described for Step I-B, to give a compound of Formula $A^5$.

REACTION SCHEME VI

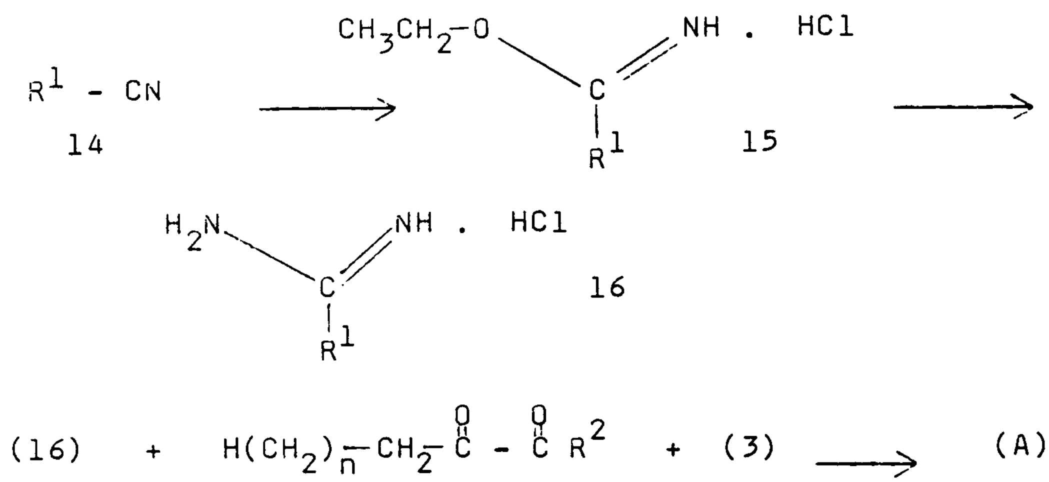

14

15

16

(16) (3) (A)

Referring to Reaction Scheme VI, the nitrile of Formula 14 is dissolved in alcohol, preferably methanol or ethanol, more preferably 99% ethanol, the solution is saturated with dry HCl gas and stirred at room temperature for from 1 to 24 hours, preferably overnight. The precipitate (compound of Formula 15) is filtered, washed and dried. A second crop of precipitate is collected by placing the mother liquors in a freezer overnight, and the precipitate is washed and dried.

Compound of Formula 15 is added slowly with stirring to an alcohol, preferably methanol, which is saturated with ammonia. Once the compound is dissolved, it is stirred at room temperature for from 1 to 24 hours, preferably overnight. The solution is reduced by 2/3 volume under vacuum, then diluted with 3 times its volume with isopropylacetate. The precipitate formed (compound of Formula 16) is filtered and dried.

Compound of Formula 16 is then dissolved in alcohol, preferably methanol or ethanol, more preferably 99% ethanol. Compound of Formula 17 is added dropwise and the reaction mixture refluxed for 1 to 40 hours, preferably 15 to 25 hours, more preferably about 20 hours. The mixture is then cooled to 15 to 40 °C, preferably about 30 °C, compound of Formula 3 is added, then water, a base (such as sodium hydroxide, potassium hydroxide, sodium carbonate, sodium bicarbonate, potassium carbonate, potassium bicarbonate, and the like, preferably sodium hydroxide) and a metal hydride (such as lithium bromide, potassium bromide, preferably lithium bromide) are added. Any insoluble material is removed by filtration and the solution is refluxed for 1 to 10 hours, preferably 4 to 6 hours. The reaction mixture is cooled and stirred at room temperature for 1 to 24 hours, preferably overnight. The precipitate is filtered, then poured into an alcohol/water mixture (such as methanol/water, ethanol/water, isopropanol/water in a ratio of from 10:90 to about 90:10, preferably 60:40). The solution is warmed to 50 to 80 °C, preferably about 70 °C, for 15 minutes to 4 hours, preferably about 1 hour, then cooled, and the precipitate filtered and dried to yield compound of Formula A.

## Preferred Processes

The compounds of the present invention can be prepared according to any of the following last steps, in which non-essential substituents are not referenced, but will be apparent from reference to the foregoing reaction schemes:

a 4-haloalkyl-1H-imidazole (or a salt thereof) is condensed with a substituted-4-piperazine to give a compound according to Formula A where m is 2;

a 4-haloalkyl-1H-imidazole (or a salt thereof) is condensed with a substituted-4-diazepine to give a compound according to Formula A where m is 3;

a 4-(1-halo-$C_2$-$C_4$-alkyl)-1H-imidazole (or a salt thereof) is condensed with a substituted-4-piperazine to give a compound according to Formula A wherein m is 2, and $R^3$ is lower alkyl;

a 4-(1-halo-$C_2$-$C_4$-alkyl)-1H-imidazole (or a salt thereof) is condensed with a substituted-4-diazepine to give a compound according to Formula A wherein m is 3, and $R^3$ is lower alkyl;

a 1-substituted-4-[(1H-imidazol-4-yl)-1-oxo-alkyl]piperazine is reduced to give the corresponding compound according to Formula A where m is 2, and $R^3$ is hydroxy;

a 1-substituted-4-[(1H-imidazol-4-yl)-1-oxo-alkyl]diazepine is reduced to give the corresponding compound according to Formula A where m is 3, and $R^3$ is hydroxy;

a 2-(aryl)-4-(ω-haloalkyl)-5-alkyl-1H-imidazole (or a salt thereof) is condensed with a diaryl-alkyl-4-piperazine to give a compound according to Formula A where $R^1$ is aryl, $R^2$ is alkyl, $R^3$ is hydrogen, $R^4$ and $R^5$ are aryl, m is 2, n is 0-2, and q is 0-3;

a substituted amidine is reacted with a substituted dione and a substituted-4-piperazine or a substituted-4-diazepine to give a compound of Formula A;

a pharmaceutically acceptable acid is reacted with a compound of Formula A to form the corresponding acid addition salt of Formula A;

a pharmaceutically acceptable acid addition salt of Formula A is converted to another pharmaceutically acceptable acid addition salt of Formula A; or

an acid addition salt of Formula A is reacted with a base to form the corresponding free base compound of Formula A.

## PREPARATION 1

Preparation of Compound of Formula 1

A solution of 1 mole (117.5 g) of tolunitrile in 500 ml absolute ethanol (99%) was saturated with dry HCl gas then kept under stirring at room temperature overnight. A first crop of crystals was collected by filtration then mother liquors were concentrated and placed in a freezer (-18°C) for 16 hours. A second crop was obtained, giving an overall yield of 80%. Without further purification, the crude product (160 g) was put in 200 ml $NH_3$-saturated methanol, portionwise with stirring. After complete dissolution the reaction medium was left at room temperature overnight then 1/3 of the methanol was removed under reduced pressure. The resulting solution was diluted with 200 ml of isopropyl- acetate and left to crystallize at room temperature for one day. The desired toluamidine hydrochloride was recovered by filtration, dried overnight at 50°C and used without further purification in the next step (77 g of a white powder, 90% yield).

77 g of the above benzamidine hydrochloride were dissolved in 200 ml of water then one equivalent of butanedione was added. The pH was adjusted to 7 with 2N sodium hydroxide and the reaction medium was left for two hours at 0°C. The white solid formed was filtered off and washed with acetone. The crude product (93 g) was dissolved in 500 ml of 10N HCl then the solution was heated to 100°C with stirring for 6 hours. After cooling, the white solid formed was recovered by filtration and recrystallized from water to give 65 g of 2-(4-methylphenyl)-5-methyl-4-hydroxymethyl-1H-imidazole, melting point, 167-169°C.

This 4-hydroxymethyl-1H-imidazole was then reacted with a thionyl halide using the procedures set forth above to form the 2-(4-methylphenyl)-4-chloromethyl-5-methyl-1H-imidazole hydrochloride used in Example 1.

## EXAMPLES

The following examples are given to enable those skilled in the art to more clearly understand and to practice the present invention. They should not be considered as a limitation on the scope of the invention, but merely as being illustrative and representative thereof.

## EXAMPLE 1

### 1-Diphenylmethyl-4-[(2-(4-methylphenyl)-5-methyl-1H-imidazol-4-yl)methyl]piperazine and Derivatives Thereof

### 1A. Formula A Where $R^1$ is 4-Methylphenyl; $R^2$ is Methyl; $R^3$ is Hydrogen; $R^4$ and $R^5$ are Phenyl; m is 2; n is 0; and q is 0

50 Grams (0.2 mol) of 2-(4-methylphenyl)-4-chloromethyl-5-methyl-1H-imidazole hydrochloride dissolved in 200 ml of a mixture of ethanol:water 60:40 were added dropwise to a refluxing solution of 55 grams (0.2 mol) of N-(diphenylmethyl)piperazine and 24 grams (0.6 mol) sodium hydroxide in 200 ml of a mixture of ethanol:water 60:40. After 2 to 3 hours 1-diphenylmethyl-4-[(2-(4-methyl-phenyl)-5-methyl-1H-imidazole-4-yl)methyl]piperazine precipitated from the reaction mixture. After having left the crystals

standing at room temperature, they were removed by filtration and recrystallized from methanol to give the free base in 70% yield which melted at 220-222 ° C.

The free base was converted to its acid addition salt by the process taught in Example 6.

1B. Formula A Varying $R^1$ and $R^2$

Similarly, following the procedure of Part A above, but replacing 2-(4-methylphenyl)-4-chloromethyl-5-methyl-1H-imidazole hydrochloride with:

2-phenyl-4-chloromethyl-5-methyl-1H-imidazole hydrochloride;
2-(3-methylphenyl)-4-chloromethyl-5-methyl-1H-imidazole hydrochloride;
2-(2-methylphenyl)-4-chloromethyl-5-methyl-1H-imidazole hydrochloride;
2-(4-t-butylphenyl)-4-chloromethyl-5-methyl-1H-imidazole hydrochloride;
2-(3-t-butylphenyl)-4-chloromethyl-5-methyl-1H-imidazole hydrochloride;
2-(2-t-butylphenyl)-4-chloromethyl-5-methyl-1H-imidazole hydrochloride;
2-(4-chlorophenyl)-4-chloromethyl-5-methyl-1H-imidazole hydrochloride;
2-(3-chlorophenyl)-4-chloromethyl-5-methyl-1H-imidazole hydrochloride;
2-(2-chlorophenyl)-4-chloromethyl-5-methyl-1H-imidazole hydrochloride;
2-(4-methoxyphenyl)-4-chloromethyl-5-methyl-1H-imidazole hydrochloride;
2-(3-methoxyphenyl)-4-chloromethyl-5-methyl-1H-imidazole hydrochloride;
2-(2-methoxyphenyl)-4-chloromethyl-5-methyl-1H-imidazole hydrochloride;
2-(2-methylphenyl)-4-chloromethyl-5-ethyl-1H-imidazole hydrochloride;
2-(4-methylphenyl)-4-chloromethyl-5-t-butyl-1H-imidazole hydrochloride;
2-(4-methylphenyl)-4-chloromethyl-1H-imidazole hydrochloride;
2-(3,4-dimethoxyphenyl)-4-bromomethyl-5-methyl-1H-imidazole hydrochloride; and
2,5-di-(4-methylphenyl)-4-chloromethyl-1H-imidazole hydrochloride;

there is obtained:

1-diphenylmethyl-4-[(2-phenyl-5-methyl-1H-imidazol-4-yl)methyl]piperazine, the trihydrochloride salt of which has a melting point of 214 ° C;
1-diphenylmethyl-4-[(2-(3-methylphenyl)-5-methyl-1H-imidazol-4-yl)methyl]piperazine;
1-diphenylmethyl-4-[(2-(2-methylphenyl)-5-methyl-1H-imidazol-4-yl)methyl]piperazine;
1-diphenylmethyl-4-[(2-(4-t-butylphenyl)-5-methyl-1H-imidazol-4-yl)methyl]piperazine;
1-diphenylmethyl-4-[(2-(3-t-butylphenyl)-5-methyl-1H-imidazol-4-yl)methyl]piperazine;
1-diphenylmethyl-4-[(2-(2-t-butylphenyl)-5-methyl-1H-imidazol-4-yl)methyl]piperazine;
1-diphenylmethyl-4-[(2-(4-chlorophenyl)-5-methyl-1H-imidazol-4-yl)methyl]piperazine, the trihydrochloride salt of which has a melting point of 216 ° C;
1-diphenylmethyl-4-[(2-(3-chlorophenyl)-5-methyl-1H-imidazol-4-yl)methyl]piperazine, the trihydrochloride salt of which has a melting point of about 215 ° C;
1-diphenylmethyl-4-[(2-(2-chlorophenyl)-5-methyl-1H-imidazol-4-yl)methyl]piperazine;
1-diphenylmethyl-4-[(2-(4-methoxyphenyl)-5-methyl-1H-imidazol-4-yl)methyl]piperazine, the trihydrochloride salt of which has a melting point of about 225 ° C;
1-diphenylmethyl-4-[(2-(3-methoxyphenyl)-5-methyl-1H-imidazol-4-yl)methyl]piperazine;
1-diphenylmethyl-4-[(2-(2-methoxyphenyl)-5-methyl-1H-imidazol-4-yl)methyl]piperazine;
1-diphenylmethyl-4-[(2-(2-methylphenyl)-5-ethyl-1H-imidazol-4-yl)methyl]piperazine;
1-diphenylmethyl-4-[(2-(4-methylphenyl)-5-t-butyl-1H-imidazol-4-yl)methyl]piperazine;
1-diphenylmethyl-4-[(2-(4-methylphenyl)-1H-imidazol-4-yl)methyl]piperazine;
1-diphenylmethyl-4-[(2-(3,4-dimethoxyphenyl)-5-methyl-1H-imidazol-4-yl)methyl]piperazine, the trihydrochloride salt of which has a melting point of 230 ° C; and
1-diphenylmethyl-4-[(2,5-di-(4-methylphenyl)-1H-imidazol-4-yl)methyl]piperazine;

1C. Formula A Varying q, $R^4$ and $R^5$

Similarly, following the procedure of Part A above, but replacing N-(diphenylmethyl)piperazine with:

N-[di-(2-methylphenyl)methyl]piperazine;
N-[di-(3-methylphenyl)methyl]piperazine;
N-[di-(4-methylphenyl)methyl]piperazine;
N-[di-(2-t-butylphenyl)methyl]piperazine;
N-[di-(3-t-butylphenyl)methyl]piperazine;
N-[di-(4-t-butylphenyl)methyl]piperazine;

N-[di-(2-methoxyphenyl)methyl]piperazine;
N-[di-(3-methoxyphenyl)methyl]piperazine;
N-[di-(4-methoxyphenyl)methyl]piperazine;
N-[di-(2-chlorophenyl)methyl]piperazine;
N-[di-(3-chlorophenyl)methyl]piperazine;
N-[di-(4-chlorophenyl)methyl]piperazine;
N-[di-(4-fluorophenyl)methyl]piperazine;
N-[1-(4-chlorophenyl)-1-(phenyl)methyl]piperazine;
N-(2,2-diphenylethyl)piperazine;
N-[3-(phenyl)-3-(4-methoxyphenyl)propyl]piperazine;
and
N-(4,4-diphenylbutyl)piperazine,
there is obtained:
1-[di-(2-methylphenyl)methyl]-4-[(2-(4-methylphenyl)-5-methyl-1H-imidazol-4-yl)methyl]piperazine;
1-[di-(3-methylphenyl)methyl]-4-[(2-(4-methylphenyl)-5-methyl-1H-imidazol-4-yl)methyl]piperazine;
1-[di-(4-methylphenyl)methyl]-4-[(2-(4-methylphenyl)-5-methyl-1H-imidazol-4-yl)methyl]piperazine;
1-[di-(2-t-butylphenyl)methyl]-4-[(2-(4-methylphenyl)-5-methyl-1H-imidazol-4-yl)methyl]piperazine;
1-[di-(3-t-butylphenyl)methyl]-4-[(2-(4-methylphenyl)-5-methyl-1H-imidazol-4-yl)methyl]piperazine;
1-[di-(4-t-butylphenyl)methyl]-4-[(2-(4-methylphenyl)-5-methyl-1H-imidazol-4-yl)methyl]piperazine;
1-[di-(2-methoxyphenyl)methyl]-4-[(2-(4-methylphenyl)-5-methyl-1H-imidazol-4-yl)methyl]piperazine;
1-[di-(3-methoxyphenyl)methyl]-4-[(2-(4-methylphenyl)-5-methyl-1H-imidazol-4-yl)methyl]piperazine;
1-[di-(4-methoxyphenyl)methyl]-4-[(2-(4-methylphenyl)-5-methyl-1H-imidazol-4-yl)methyl]piperazine;
1-[di-(2-chlorophenyl)methyl]-4-[(2-(4-methylphenyl)-5-methyl-1H-imidazol-4-yl)methyl]piperazine;
1-[di-(3-chlorophenyl)methyl]-4-[(2-(4-methylphenyl)-5-methyl-1H-imidazol-4-yl)methyl]piperazine;
1-[di-(4-chlorophenyl)methyl]-4-[(2-(4-methylphenyl)-5-methyl-1H-imidazol-4-yl)methyl]piperazine, the trihydrochloride salt of which has a melting point of about 225 °C;
1-[di-(4-fluorophenyl)methyl]-4-[(2-(4-methylphenyl)-5-methyl-1H-imidazol-4-yl)methyl]piperazine, the trihydrochloride salt of which has a melting point of about 210 °C;
1-[1-(4-chlorophenyl)-1-(phenyl)methyl]-4-[(2-(4-methylphenyl)-5-methyl-1H-imidazol-4-yl)methyl]-piperazine;
1-(2,2-diphenylethyl)-4-[(2-(4-methylphenyl)-5-methyl-1H-imidazol-4-yl)methyl]piperazine;
1-[3-(phenyl)-3-(4-methoxyphenyl)propyl]-4-[(2-(4-methylphenyl)-5-methyl-1H-imidazol-4-yl)methyl]-piperazine; and
1-(4,4-diphenylbutyl)-4-[(2-(4-methylphenyl)-5-methyl-1H-imidazol-4-yl)methyl]piperazine.

### 1D. Formula A Varying $R^1$; $R^2$; $R^4$; $R^5$ and q

Similarly, by following the procedures of Parts B and C above, other compounds of Formula A where $R^3$ is hydrogen, m is 2, and n is 0 are obtained, such as:

1-diphenylmethyl-4-[(2-(3-trifluoromethylphenyl)-5-methyl-1H-imidazol-4-yl)methyl]piperazine, the trihydrochloride salt of which has a melting point of about 210 °C;

1-diphenylmethyl-4-[(2-phenyl-1H-imidazol-4-yl)methyl]piperazine, the fumarate salt of which has a melting point of 170 °C;

1-[di-(4-chlorophenyl)methyl]-4-[(2-phenyl-5-methyl-1H-imidazol-4-yl)methyl]piperazine, the trihydrochloride salt of which has a melting point of 220 °C; and

1-[4,4-di-(4-fluorophenyl)butyl]-4-[(2-phenyl-5-methyl-1H-imidazol-4-yl)methyl]piperazine, the trihydrochloride salt of which has a melting point of 198 °C.

### 1E. Formula A Varying m

Similarly, by following the procedures of Parts A to D above, but replacing the piperazines there-used with the corresponding diazepines, the compounds of Formula A wherein m is 3 are obtained.

For example, substituting 2-phenyl-4-chloromethyl-5-methyl-1H-imidazolehydrochloride for 2-(4-methylphenyl)-4-chloromethyl-5-methyl-1H-imidazole hydrochloride, and by substituting diphenylmethyl-4-diazepine for diphenylmethyl-4-piperazine, there is obtained 1-diphenylmethyl-4-[(2-phenyl-5-methyl-1H-imidazol-4-yl)methyl]diazepine, the trihydrochloride salt of which has a melting point of about 205 °C.

## EXAMPLE 2

1-Diphenylmethyl-4-[1-(2-phenyl-5-methyl-1H-imidazol-4-yl)ethyl]piperazine and Derivatives Thereof

2A. Formula 5 Where $R^1$ is Phenyl; and $R^2$ is Methyl

32 Grams (0.59 mol) of potassium borohydride were added portionwise to a solution of 30 g (0.15 mol) of 2-phenyl-4-acetyl-5-methylimidazole in 1500 ml of MeOH. After stirring overnight, a solid material was removed by filtration, then the solvent was evaporated under reduced pressure to give 27 g of 2-phenyl-4-(1-hydroxyethyl)-5-methylimidazole. The crude compound thus isolated was used without further purification.

2B. Formula 6 Where $R^1$ is Phenyl; $R^2$ is Methyl; and X is Chloro

27 Grams (0.13 mol) of 2-phenyl-4-(1-hydroxyethyl)-5-methylimidazole were dissolved in 700 ml of chloroform with 44 ml (0.6 mol) of thionyl chloride and refluxed for 5 hours. After cooling, the mixture was evaporated, the residue triturated in acetone, thereby giving, in approximately stoichiometric yield, 2-phenyl-4-(1-chloroethyl)-5-methylimidazole hydrochloride, m.p. 190 ° C.

2C. Formula 6 Varying $R^1$, $R^2$, and the Length of Alkyl at Position 4 of the Imidazole

Similarly, following the procedures of Part A and B above, but replacing 2-phenyl-4-acetyl-5-methylimidazole with:

2-(phenyl)-4-(2-methylpropanoyl)-5-methylimidazole;
2-(3-methylphenyl)-4-acetyl-5-methylimidazole;
2-(2-methylphenyl)-4-acetyl-5-methylimidazole;
2-(4-t-butylphenyl)-4-acetyl-5-methylimidazole;
2-(3-t-butylphenyl)-4-acetyl-5-methylimidazole;
2-(2-t-butylphenyl)-4-acetyl-5-methylimidazole;
2-(4-chlorophenyl)-4-acetyl-5-methylimidazole;
2-(3-chlorophenyl)-4-acetyl-5-methylimidazole;
2-(2-chlorophenyl)-4-acetyl-5-methylimidazole;
2-(4-methoxyphenyl)-4-acetyl-5-methylimidazole;
2-(3-methoxyphenyl)-4-acetyl-5-methylimidazole;
2-(2-methoxyphenyl)-4-acetyl-5-methylimidazole;
2-(2-methylphenyl)-4-acetyl-5-ethylimidazole;
2-(4-methylphenyl)-4-acetyl-5-t-butylimidazole; and
2-(4-methylphenyl)-4-acetylimidazole,

there is obtained:

2-(phenyl)-4-(1-chloro-2-methylpropyl)-5-methylimidazole hydrochloride;
2-(3-methylphenyl)-4-(1-chloroethyl)-5-methylimidazole hydrochloride;
2-(2-methylphenyl)-4-(1-chloroethyl)-5-methylimidazole hydrochloride;
2-(4-t-butylphenyl)-4-(1-chloroethyl)-5-methylimidazole hydrochloride;
2-(3-t-butylphenyl)-4-(1-chloroethyl)-5-methylimidazole hydrochloride;
2-(2-t-butylphenyl)-4-(1-chloroethyl)-5-methylimidazole hydrochloride;
2-(4-chlorophenyl)-4-(1-chloroethyl)-5-methylimidazole hydrochloride;
2-(3-chlorophenyl)-4-(1-chloroethyl)-5-methylimidazole hydrochloride;
2-(2-chlorophenyl)-4-(1-chloroethyl)-5-methylimidazole hydrochloride;
2-(4-methoxyphenyl)-4-(1-chloroethyl)-5-methylimidazole hydrochloride;
2-(3-methoxyphenyl)-4-(1-chloroethyl)-5-methylimidazole hydrochloride;
2-(2-methoxyphenyl)-4-(1-chloroethyl)-5-methylimidazole hydrochloride;
2-(2-methylphenyl)-4-(1-chloroethyl)-5-ethylimidazole hydrochloride;
2-(4-methylphenyl)-4-(1-chloroethyl)-5-t-butylimidazole hydrochloride; and
2-(4-methylphenyl)-4-(1-chloroethyl)-imidazole hydrochloride.

2D. Formula A Where $R^1$ is Phenyl; $R^2$ is Methyl; $R^3$ is Methyl; $R^4$ and $R^5$ are Phenyl; m is 2; n is 0; and q is 0

14 Grams (0.052 mol) of N-(diphenylmethyl)piperazine and 6 grams (0.15 mol) of sodium hydroxide were dissolved in 180 ml of a mixture of ethanol:water 60:40. The mixture was heated to reflux, then 2-phenyl-4-(1-chloroethyl)-5-methylimidazole hydrochloride in 180 milliliters of ethanol:water 60:40 were added dropwise. After 4 to 5 hours under reflux, the reaction mixture was allowed to cool to room temperature. The oil that separated was washed twice with water, then dissolved in ether and hydrochloric acid was added. The precipitate was recrystallized from ethanol to give 1-diphenylmethyl-4-[1-(2-phenyl-5-methyl-1H-imidazol-4-yl)ethyl]piperazine trihydrochloride (55% yield), which melted at 215 °C.

2E. Formula A Where $R^3$ is Methyl; $R^4$ and $R^5$ are Phenyl; m is 2; n is 0; q is 0; and Varying $R^1$ and $R^2$

Similarly, following the procedure of Part D above, but replacing 2-phenyl-4-(1-chloroethyl)-5-methylimidazole hydrochloride with:

2-(phenyl)-4-(1-chloro-2-methylpropyl)-5-methylimidazole hydrochloride;
2-(phenyl)-4-(1-chloroethyl)-5-methylimidazole hydrochloride;
2-(3-methylphenyl)-4-(1-chloroethyl)-5-methylimidazole hydrochloride;
2-(2-methylphenyl)-4-(1-chloroethyl)-5-methylimidazole hydrochloride;
2-(4-t-butylphenyl)-4-(1-chloroethyl)-5-methylimidazole hydrochloride;
2-(3-t-butylphenyl)-4-(1-chloroethyl)-5-methylimidazole hydrochloride;
2-(2-t-butylphenyl)-4-(1-chloroethyl)-5-methylimidazole hydrochloride;
2-(4-chlorophenyl)-4-(1-chloroethyl)-5-methylimidazole hydrochloride;
2-(3-chlorophenyl)-4-(1-chloroethyl)-5-methylimidazole hydrochloride;
2-(2-chlorophenyl)-4-(1-chloroethyl)-5-methylimidazole hydrochloride;
2-(4-methoxyphenyl)-4-(1-chloroethyl)-5-methylimidazole hydrochloride;
2-(3-methoxyphenyl)-4-(1-chloroethyl)-5-methylimidazole hydrochloride;
2-(2-methoxyphenyl)-4-(1-chloroethyl)-5-methylimidazole hydrochloride;
2-(2-methylphenyl)-4-(1-chloroethyl)-5-ethylimidazole hydrochloride;
2-(4-methylphenyl)-4-(1-chloroethyl)-5-t-butylimidazole hydrochloride; and
2-(4-methylphenyl-4-(1-chloroethyl)-imidazole hydrochloride,

there is obtained:

1-diphenylmethyl-4-[1-(2-phenyl-5-methyl-1H-imidazol-4-yl)propyl]piperazine hydrochloride;
1-diphenylmethyl-4-[1-(2-phenyl-5-methyl-1H-imidazol-4-yl)ethyl]piperazine trihydrochloride;
1-diphenylmethyl-4-[1-(2-(3-methylphenyl)-5-methyl-1H-imidazol-4-yl)ethyl]piperazine trihydrochloride;
1-diphenylmethyl-4-[1-(2-(2-methylphenyl)-5-methyl-1H-imidazol-4-yl)ethyl]piperazine trihydrochloride;
1-diphenylmethyl-4-[1-(2-(4-t-butylphenyl)-5-methyl-1H-imidazol-4-yl)ethyl]piperazine trihydrochloride;
1-diphenylmethyl-4-[1-(2-(3-t-butylphenyl)-5-methyl-1H-imidazol-4-yl)ethyl]piperazine trihydrochloride;
1-diphenylmethyl-4-[1-(2-(2-t-butylphenyl)-5-methyl-1H-imidazol-4-yl)ethyl]piperazine trihydrochloride;
1-diphenylmethyl-4-[1-(2-(4-chlorophenyl)-5-methyl-1H-imidazol-4-yl)ethyl]piperazine trihydrochloride;
1-diphenylmethyl-4-[1-(2-(3-chlorophenyl)-5-methyl-1H-imidazol-4-yl)ethyl]piperazine trihydrochloride;
1-diphenylmethyl-4-[1-(2-(2-chlorophenyl)-5-methyl-1H-imidazol-4-yl)ethyl]piperazine trihydrochloride;
1-diphenylmethyl-4-[1-(2-(4-methoxyphenyl)-5-methyl-1H-imidazol-4-yl)ethyl]piperazine trihydrochloride;
1-diphenylmethyl-4-[1-(2-(3-methoxyphenyl)-5-methyl-1H-imidazol-4-yl)ethyl]piperazine trihydrochloride;
1-diphenylmethyl-4-[1-(2-(2-methoxyphenyl)-5-methyl-1H-imidazol-4-yl)ethyl]piperazine trihydrochloride;
1-diphenylmethyl-4-[1-(2-(2-methylphenyl)-5-ethyl-1H-imidazol-4-yl)ethyl]piperazine trihydrochloride;
1-diphenylmethyl-4-[1-(2-(4-methylphenyl)-5-t-butyl-1H-imidazol-4-yl)ethyl]piperazine trihydrochloride; and

1-diphenylmethyl-4-[1-(2-(4-methylphenyl)-1H-imidazol-4-yl)ethyl]piperazine trichydrochloride.

2F. Formula A Where $R^1$ is Phenyl; $R^2$ is Methyl; $R^3$ is Methyl; m is 2; n is 0; and Varying q, $R^4$ and $R^5$

Similarly, following the procedure of Part D above, but replacing N-(diphenylmethyl)piperazine with:

N-[di-(2-methylphenyl)methyl]piperazine;
N-[di-(3-methylphenyl)methyl]piperazine;
N-[di-(4-methylphenyl)methyl]piperazine;
N-[di-(2-t-butylphenyl)methyl]piperazine;
N-[di-(3-t-butylphenyl)methyl]piperazine;
N-[di-(4-t-butylphenyl)methyl]piperazine;
N-[di-(2-methoxyphenyl)methyl]piperazine;
N-[di-(3-methoxyphenyl)methyl]piperazine;
N-[di-(4-methoxyphenyl)methyl]piperazine;
N-[di-(2-chlorophenyl)methyl]piperazine;

N-[di-(3-chlorophenyl)methyl]piperazine;
N-[di-(4-chlorophenyl)methyl]piperazine;
N-[di-(4-fluorophenyl)methyl]piperazine;
N-[1-(4-chlorophenyl)-1-(phenyl)methyl]piperazine;
N-(2,2-diphenylethyl)piperazine;
N-[3-(phenyl)-3-(4-methoxyphenyl)propyl]piperazine;
and
N-(4,4-diphenylbutyl)piperazine,
there is obtained:
1-[di-(2-methylphenyl)methyl]-4-[1-(2-phenyl-5-methyl-1H-imidazol-4-yl)ethyl]piperazine trihydrochloride;
1-[di-(3-methylphenyl)methyl]-4-[1-(2-phenyl-5-methyl-1H-imidazol-4-yl)ethyl]piperazine trihydrochloride;
1-[di-(4-methylphenyl)methyl]-4-[1-(2-phenyl-5-methyl-1H-imidazol-4-yl)ethyl]piperazine trihydrochloride;
1-[di-(2-t-butylphenyl)methyl]-4-[1-(2-phenyl-5-methyl-1H-imidazol-4-yl)ethyl]piperazine trihydrochloride;
1-[di-(3-t-butylphenyl)methyl]-4-[1-(2-phenyl-5-methyl-1H-imidazol-4-yl)ethyl]piperazine trihydrochloride;
1-[di-(4-t-butylphenyl)methyl]-4-[1-(2-phenyl-5-methyl-1H-imidazol-4-yl)ethyl]piperazine trihydrochloride;
1-[di-(2-methoxyphenyl)methyl]-4-[1-(2-phenyl-5-methyl-1H-imidazol-4-yl)ethyl]piperazine trihydrochloride;
1-[di-(3-methoxyphenyl)methyl]-4-[1-(2-phenyl-5-methyl-1H-imidazol-4-yl)ethyl]piperazine trihydrochloride;
1-[di-(4-methoxyphenyl)methyl]-4-[1-(2-phenyl-5-methyl-1H-imidazol-4-yl)ethyl]piperazine trihydrochloride;
1-[di-(2-chlorophenyl)methyl]-4-[1-(2-phenyl-5-methyl-1H-imidazol-4-yl)ethyl]piperazine trihydrochloride;
1-[di-(3-chlorophenyl)methyl]-4-[1-(2-phenyl-5-methyl-1H-imidazol-4-yl)ethyl]piperazine trihydrochloride;
1-[di-(4-chlorophenyl)methyl]-4-[1-(2-phenyl-5-methyl-1H-imidazol-4-yl)ethyl]piperazine trihydrochloride;
1-[di-(4-fluorophenyl)methyl]-4-[1-(2-phenyl-5-methyl-1H-imidazol-4-yl)ethyl]piperazine trihydrochloride;
1-[(4-chlorophenyl)-1-(phenyl)methyl]-4-[1-(2-phenyl-5-methyl-1H-imidazol-4-yl)ethyl]piperazine trihydrochloride;
1-(2,2-diphenylethyl)-4-[1-(2-phenyl-5-methyl-1H-imidazol-4-yl)ethyl]piperazine trihydrochloride;
1-[3-(phenyl)-3-(4-methoxyphenyl)propyl]-4-[1-(2-phenyl-5-methyl-1H-imidazol-4-yl)ethyl]piperazine trihydrochloride; and
1-(4,4-diphenylbutyl)-4-[1-(2-phenyl-5-methyl-1H-imidazol-4-yl)ethyl]piperazine trihydrochloride.

2G. Formula A Where $R^3$ is Methyl; m is 2; n is 0; and Varying $R^1$; $R^2$; $R^4$; $R^5$ and q

Similarly, by following the procedures of Parts E and F above, other compounds of Formula A where $R^3$ is methyl, m is 2, and n is 0 are obtained, such as:
1-[(4-chlorophenyl)-1-(phenyl)methyl]-4-[1-(2-phenyl-5-(4-methylphenyl)-1H-imidazol-4-yl)ethyl]-piperazine trihydrochloride; and
1-[3-(phenyl)-3-(4-methoxyphenyl)propyl]-4-[1-(2-(3-methoxyphenyl)-5-propyl-1H-imidazol-4-yl)ethyl]-piperazine trihydrochloride.

2H. Formula A Where $R^3$ is Lower Alkyl Other Than Methyl

Similarly, following the procedure of Part D above, but replacing 2-phenyl-4-(1-chloroethyl)-5-methylimidazole hydrochloride with:
2-phenyl-4-(1-chloropropyl)-5-methylimidazole hydrochloride; and
2-phenyl-4-(1-chlorobutyl)-5-methylimidazole hydrochloride,
there is obtained:
1-diphenylmethyl-4-[1-(2-phenyl-5-methyl-1H-imidazol-4-yl)propyl]piperazine trihydrochloride; and
1-diphenylmethyl-4-[1-(2-phenyl-5-methyl-1H-imidazol-4-yl)butyl]piperazine trihydrochloride.

2I. Formula A Varying m

Similarly, by following the procedures of Parts A-H above, but replacing the piperazines there-used with the corresponding diazepines, the compounds of Formula A wherein m is 3 are obtained.

EXAMPLE 3

1-Diphenylmethyl-4-[2-(2-phenyl-5-methyl-1H-imidazol-4-yl)-2-hydroxyethyl]piperazine trihydrochloride and Derivatives Thereof

3A. Formula 9 Where $R^1$ is Phenyl; $R^2$ is Methyl; $R^4$ and $R^5$ are Phenyl; m is 2; and q is 0

10 Grams (0.036 mol) of 2-phenyl-4-(2-bromoethanoyl)-5-methyl-1H-imidazole and 8.5 grams (0.034 mol) of N-(diphenylmethyl)piperazine and 5 grams (0.036 mol) of potassium carbonate were added to 300 ml of ethanol. The mixture was refluxed under stirring overnight. After cooling, the salts were removed by filtration and the solvent was removed under reduced pressure. The residue was extracted by dichloromethane and washed twice with water. The organic layer was dried over sodium sulfate and evaporated. Trituration of the residue with ethanol gave a white precipitate, 1-diphenylmethyl-4-[2-(2-phenyl-5-methyl-1H-imidazol-4-yl)-2-oxoethyl]piperazine, which was used in the next reaction step without further purification.

3B. TriHCl Salt of Formula A Where $R^1$ is Phenyl; $R^2$ is Methyl; $R^3$ is Hydroxy; $R^4$ and $R^5$ are Phenyl; m is 2; n is 1; and q is 0

6 Grams of 1-diphenylmethyl-4-[2-(2-phenyl-5-methyl-1H-imidazol-4-yl)-2-oxoethyl]piperazine was dissolved in 100 ml of methanol. The reaction was cooled to 5°C and then 2 grams (0.05 mol) of sodium borohydride was added portionwise. After stirring for 2 hours at room temperature, the mixture was evaporated off. The residue was extracted with dichloromethane and washed with water. Then the organic layer was dried over sodium sulphate and the solvent removed under reduced pressure. The crude material was then dissolved in diethyloxide and hydrochloric acid was added. The white precipitate was then removed by filtration and dried to give 1-diphenylmethyl-4-[2-(2-phenyl-5-methyl-1H-imidazol-4-yl)-2-hydroxyethyl]piperazine trihydrochloride, which melted at 200°C.

3C. TriHCl Salt of Formula A Where $R^3$ is Hydroxy; $R^4$ and $R^5$ are Phenyl; m is 2; n is 1; q is 0; and Varying $R^1$ and $R^2$

Similarly, following the procedures of Parts A and B above, but replacing 2-phenyl-4-(2-bromoethanoyl)-5-methyl-1H-imidazole with:

2-(3-methylphenyl)-4-(2-bromoethanoyl)-5-methyl-1H-imidazole;
2-(2-methylphenyl)-4-(2-bromoethanoyl)-5-methyl-1H-imidazole;
2-(4-t-butylphenyl)-4-(2-bromoethanoyl)-5-methyl-1H-imidazole;
2-(3-t-butylphenyl)-4-(2-bromoethanoyl)-5-methyl-1H-imidazole;
2-(2-t-butylphenyl)-4-(2-bromoethanoyl)-5-methyl-1H-imidazole;
2-(4-chlorophenyl)-4-(2-bromoethanoyl)-5-methyl-1H-imidazole;
2-(3-chlorophenyl)-4-(2-bromoethanoyl)-5-methyl-1H-imidazole;
2-(2-chlorophenyl)-4-(2-bromoethanoyl)-5-methyl-1H-imidazole;
2-(4-methoxyphenyl)-4-(2-bromoethanoyl)-5-methyl-1H-imidazole;
2-(3-methoxyphenyl)-4-(2-bromoethanoyl)-5-methyl-1H-imidazole;
2-(2-methoxyphenyl)-4-(2-bromoethanoyl)-5-methyl-1H-imidazole;
2-(2-methylphenyl)-4-(2-bromoethanoyl)-5-ethyl-1H-imidazole;
2-(4-methylphenyl)-4-(2-bromoethanoyl)-5-t-butyl-1H-imidazole; and
2-(4-methylphenyl)-4-(2-bromoethanoyl)-1H-imidazole, there is obtained:

1-diphenylmethyl-4-[2-(2-(3-methylphenyl)-5-methyl-1H-imidazol-4-yl)-2-hydroxyethyl]piperazine trihydrochloride;

1-diphenylmethyl-4-[2-(2-(2-methylphenyl)-5-methyl-1H-imidazol-4-yl)-2-hydroxyethyl]piperazine trihydrochloride;

1-diphenylmethyl-4-[2-(2-(4-t-butylphenyl)-5-methyl-1H-imidazol-4-yl)-2-hydroxyethyl]piperazine trihydrochloride;

1-diphenylmethyl-4-[2-(2-(3-t-butylphenyl)-5-methyl-1H-imidazol-4-yl)-2-hydroxyethyl]piperazine trihydrochloride;

1-diphenylmethyl-4-[2-(2-(2-t-butylphenyl)-5-methyl-1H-imidazol-4-yl)-2-hydroxyethyl]piperazine trihydrochloride;

1-diphenylmethyl-4-[2-(2-(4-chlorophenyl)-5-methyl-1H-imidazol-4-yl)-2-hydroxyethyl]piperazine trihydrochloride;

1-diphenylmethyl-4-[2-(2-(3-chlorophenyl)-5-methyl-1H-imidazol-4-yl)-2-hydroxyethyl]piperazine trihydrochloride;

1-diphenylmethyl-4-[2-(2-(2-chlorophenyl)-5-methyl-1H-imidazol-4-yl)-2-hydroxyethyl]piperazine trihydrochloride;

1-diphenylmethyl-4-[2-(2-(4-methoxyphenyl)-5-methyl-1H-imidazol-4-yl)-2-hydroxyethyl]piperazine trihydrochloride;

1-diphenylmethyl-4-[2-(2-(3-methoxyphenyl)-5-methyl-1H-imidazol-4-yl)-2-hydroxyethyl]piperazine trihydrochloride;

1-diphenylmethyl-4-[2-(2-(2-methoxyphenyl)-5-methyl-1H-imidazol-4-yl)-2-hydroxyethyl]piperazine trihydrochloride;

1-diphenylmethyl-4-[2-(2-(2-methylphenyl)-5-ethyl-1H-imidazol-4-yl)-2-hydroxyethyl]piperazine trihydrochloride;

1-diphenylmethyl-4-[2-(2-(4-methylphenyl)-5-t-butyl-1H-imidazol-4-yl)-2-hydroxyethyl]piperazine trihydrochloride; and

1-diphenylmethyl-4-[2-(2-(4-methylphenyl)-1H-imidazol-4-yl)-2-hydroxyethyl]piperazine trihydrochloride.

3D. TriHCl Salt of Formula A Where $R^1$ is Phenyl; $R^2$ is Methyl; $R^3$ is Hydroxy; m is 2; n is 1; q is 0; and Varying $R^4$ and $R^5$

Similarly, following the procedures of Parts A and B above, but replacing N-(diphenylmethyl)piperazine with:

N-[di-(2-methylphenyl)methyl]piperazine;
N-[di-(3-methylphenyl)methyl]piperazine;
N-[di-(4-methylphenyl)methyl]piperazine;
N-[di-(2-t-butylphenyl)methyl]piperazine;
N-[di-(3-t-butylphenyl)methyl]piperazine;
N-[di-(4-t-butylphenyl)methyl]piperazine;
N-[di-(2-methoxyphenyl)methyl]piperazine;
N-[di-(3-methoxyphenyl)methyl]piperazine;
N-[di-(4-methoxyphenyl)methyl]piperazine;
N-[di-(2-chlorophenyl)methyl]piperazine;
N-[di-(3-chlorophenyl)methyl]piperazine;
N-[di-(4-chlorophenyl)methyl]piperazine;
N-[di-(4-fluorophenyl)methyl]piperazine;
N-[1-(4-chlorophenyl)-1-(phenyl)methyl]piperazine;
N-(2,2-diphenylethyl)piperazine;
N-[3-(phenyl)-3-(4-methoxyphenyl)propyl]piperazine; and
N-(4,4-diphenylbutyl)piperazine, there is obtained:

1-[di-(2-methylphenyl)methyl]-4-[2-(2-phenyl-5-methyl-1H-imidazol-4-yl)-2-hydroxyethyl]piperazine trihydrochloride;

1-[di-(3-methylphenyl)methyl]-4-[2-(2-phenyl-5-methyl-1H-imidazol-4-yl)-2-hydroxyethyl]piperazine trihydrochloride;

1-[di-(4-methylphenyl)methyl]-4-[2-(2-phenyl-5-methyl-1H-imidazol-4-yl)-2-hydroxyethyl]piperazine trihydrochloride;

1-[di-(2-t-butylphenyl)methyl]-4-[2-(2-phenyl-5-methyl-1H-imidazol-4-yl)-2-hydroxyethyl]piperazine trihydrochloride;

1-[di-(3-t-butylphenyl)methyl]-4-[2-(2-phenyl-5-methyl-1H-imidazol-4-yl)-2-hydroxyethyl]piperazine trihydrochloride;

1-[di-(4-t-butylphenyl)methyl]-4-[2-(2-phenyl-5-methyl-1H-imidazol-4-yl)-2-hydroxyethyl]piperazine trihydrochloride;

1-[di-(2-methoxyphenyl)methyl]-4-[2-(2-phenyl-5-methyl-1H-imidazol-4-yl)-2-hydroxyethyl]piperazine trihydrochloride;

1-[di-(3-methoxyphenyl)methyl]-4-[2-(2-phenyl-5-methyl-1H-imidazol-4-yl)-2-hydroxyethyl]piperazine trihydrochloride;

1-[di-(4-methoxyphenyl)methyl]-4-[2-(2-phenyl-5-methyl-1H-imidazol-4-yl)-2-hydroxyethyl]piperazine trihydrochloride;

1-[di-(2-chlorophenyl)methyl]-4-[2-(2-phenyl-5-methyl-1H-imidazol-4-yl)-2-hydroxyethyl]piperazine trihydrochloride;

1-[di-(3-chlorophenyl)methyl]-4-[2-(2-phenyl-5-methyl-1H-imidazol-4-yl)-2-hydroxyethyl]piperazine trihydrochloride;

1-[di-(4-chlorophenyl)methyl]-4-[2-(2-phenyl-5-methyl-1H-imidazol-4-yl)-2-hydroxyethyl]piperazine trihydrochloride;

1-[di-(4-fluorophenyl)methyl]-4-[2-(2-phenyl-5-methyl-1H-imidazol-4-yl)-2-hydroxyethyl]piperazine trihydrochloride;

1-[(4-chlorophenyl)-1-(phenyl)methyl]-4-[2-(2-phenyl-5-methyl-1H-imidazol-4-yl)-2-hydroxyethyl]-piperazine trihydrochloride;

1-(2,2-diphenylethyl)-4-[2-(2-phenyl-5-methyl-1H-imidazol-4-yl)-2-hydroxyethyl]piperazine trihydrochloride;

1-[3-(phenyl)-3-(4-methoxyphenyl)propyl]-4-[2-(2-phenyl-5-methyl-1H-imidazol-4-yl)-2-hydroxyethyl]-piperazine trihydrochloride; and

1-(4,4-diphenylbutyl)-4-[2-(2-phenyl-5-methyl-1H-imidazol-4-yl)-2-hydroxyethyl]piperazine trihydrochloride.

3E. TriHCl Salt of Formula A Where $R^3$ is Hydroxy; m is 2; n is 1 or 2; and Varying $R^1$; $R^2$; $R^4$; $R^5$ and q

Similarly, by following the procedures of Parts C and D above, other compounds of Formula A where $R^3$ is hydroxy, m is 2, and n is 1 or 2 are obtained, such as:

1-[(4-chlorophenyl)-1-(phenyl)methyl]-4-[3-(2-phenyl-5-methyl-1H-imidazol-4-yl)-3-hydroxypropyl]-piperazine trihydrochloride, by starting with 2-phenyl-4-(3-bromopropanoyl)-5-methyl-1H-imidazole in part 3A; and

1-(2,2-diphenylethyl)-4-[2-(2-(4-methylphenyl)-5-methyl-1H-imidazol-4-yl)-2-hydroxyethyl]piperazine trihydrochloride;

3F. Formula A Varying m

Similarly, by following the procedures of Parts A-E above, but replacing the piperazines there-used with the corresponding diazepines, the compounds of Formula A wherein m is 3 are obtained.

## EXAMPLE 4

### 1-Diphenylmethyl-4-[1-(2-phenyl-5-methyl-1H-imidazol-4-yl)-2-methylpropyl]piperazine and Derivatives Thereof

4A. Formula 12 Where $R^1$ is Phenyl; $R^2$ is Methyl; and $R^3$ is Isopropyl

35 Grams (0.45 mol) of 2-chloropropane was added to 10.8 g (0.45 mol) of magnesium in 100 ml of diethyl ether. Then 55.8 g (0.3 mol) of 2-phenyl-4-formyl-5-methyl-1H-imidazolein 100 ml of THF were added. At the end of the addition, the mixture was refluxed for 30 minutes and then cooled and poured on ice water. The aqueous layer was extracted twice with 100 ml of diethyl ether. Evaporation of the solvent gave a residue which was recrystallized in ethanol to yield 40 grams (58% yield) of 2-phenyl-4-(1-hydroxy-2-methylpropyl)-5-methyl-1H-imidazole, which melted at 214° C.

4B. Formula 12 Where $R^6$ is Isopropyl; and Varying $R^1$ and $R^2$

Similarly, following the procedure of Part A above, but replacing 2-phenyl-4-formyl-5-methyl-1H-imidazole with:

2-(3-methylphenyl)-5-methyl-4-formyl-1H-imidazole;
2-(2-methylphenyl)-5-methyl-4-formyl-1H-imidazole;
2-(4-t-butylphenyl)-5-methyl-4-formyl-1H-imidazole;
2-(3-t-butylphenyl)-5-methyl-4-formyl-1H-imidazole;
2-(2-t-butylphenyl)-5-methyl-4-formyl-1H-imidazole;
2-(4-chlorophenyl)-5-methyl-4-formyl-1H-imidazole;
2-(3-chlorophenyl)-5-methyl-4-formyl-1H-imidazole;
2-(2-chlorophenyl)-5-methyl-4-formyl-1H-imidazole;
2-(4-methoxyphenyl)-5-methyl-4-formyl-1H-imidazole;
2-(3-methoxyphenyl)-5-methyl-4-formyl-1H-imidazole;
2-(2-methoxyphenyl)-5-methyl-4-formyl-1H-imidazole;
2-(2-methylphenyl)-5-ethyl-4-formyl-1H-imidazole;

2-(4-methylphenyl)-5-t-butyl-4-formyl-1H-imidazole;
2-(4-methylphenyl)-4-formyl-1H-imidazole;
2-(3,4-dimethoxyphenyl)-4-formyl-5-methyl-1H-imidazole; and
2,5-di-(4-methylphenyl)-4-formyl-1H-imidazole, there is obtained:
2-(3-methylphenyl)-5-methyl-4-(1-hydroxy-2-methylpropyl)-1H-imidazole;
2-(2-methylphenyl)-5-methyl-4-(1-hydroxy-2-methylpropyl)-1H-imidazole;
2-(4-t-butylphenyl)-5-methyl-4-(1-hydroxy-2-methylpropyl)-1H-imidazole;
2-(3-t-butylphenyl)-5-methyl-4-(1-hydroxy-2-methylpropyl)-1H-imidazole;
2-(2-t-butylphenyl)-5-methyl-4-(1-hydroxy-2-methylpropyl)-1H-imidazole;
2-(4-chlorophenyl)-5-methyl-4-(1-hydroxy-2-methylpropyl)-1H-imidazole;
2-(3-chlorophenyl)-5-methyl-4-(1-hydroxy-2-methylpropyl)-1H-imidazole;
2-(2-chlorophenyl)-5-methyl-4-(1-hydroxy-2-methylpropyl)-1H-imidazole;
2-(4-methoxyphenyl)-5-methyl-4-(1-hydroxy-2-methylpropyl)-1H-imidazole;
2-(3-methoxyphenyl)-5-methyl-4-(1-hydroxy-2-methylpropyl)-1H-imidazole;
2-(2-methoxyphenyl)-5-methyl-4-(1-hydroxy-2-methylpropyl)-1H-imidazole;
2-(2-methylphenyl)-5-ethyl-4-(1-hydroxy-2-methylpropyl)-1H-imidazole;
2-(4-methylphenyl)-5-t-butyl-4-(1-hydroxy-2-methylpropyl)-1H-imidazole;
2-(4-methylphenyl)-4-(1-hydroxy-2-methylpropyl)-1H-imidazole;
2-(3,4-dimethoxyphenyl)-5-methyl-4-(1-hydroxy-2-methylpropyl)-1H-imidazole; and
2,5-di-(4-methylphenyl)-4-(1-hydroxy-2-methylpropyl)-1H-imidazole.

4C. Formula 13 Where $R^1$ is Phenyl; $R^2$ is Methyl; $R^3$ is Isopropyl; and X is Chloro

27 g of 2-phenyl-4-(1-hydroxy-2-methylpropyl)-5-methyl-1H-imidazole are dissolved in 700 ml of chloroform with 44 ml of thionyl chloride ($SOCl_2$) and refluxed for 5 hours. 2-Phenyl-5-methyl-4-(1-chloro-2-methylpropyl)-1H-imidazole hydrochloride is isolated in quantitative yield.

4D. Formula 13 Where $R^6$ is Isopropyl; X is Chloro; and Varying $R^1$ and $R^2$

Similarly, following the procedure of Part C above, but replacing 2-phenyl-5-methyl-4-(1-hydroxy-2-methylpropyl)-1H-imidazole with:
2-(3-methylphenyl)-5-methyl-4-(1-hydroxy-2-methylpropyl)-1H-imidazole;
2-(2-methylphenyl)-5-methyl-4-(1-hydroxy-2-methylpropyl)-1H-imidazole;
2-(4-t-butylphenyl)-5-methyl-4-(1-hydroxy-2-methylpropyl)-1H-imidazole;
2-(3-t-butylphenyl)-5-methyl-4-(1-hydroxy-2-methylpropyl)-1H-imidazole;
2-(2-t-butylphenyl)-5-methyl-4-(1-hydroxy-2-methylpropyl)-1H-imidazole;
2-(4-chlorophenyl)-5-methyl-4-(1-hydroxy-2-methylpropyl)-1H-imidazole;
2-(3-chlorophenyl)-5-methyl-4-(1-hydroxy-2-methylpropyl)-1H-imidazole;
2-(2-chlorophenyl)-5-methyl-4-(1-hydroxy-2-methylpropyl)-1H-imidazole;
2-(4-methoxyphenyl)-5-methyl-4-(1-hydroxy-2-methylpropyl)-1H-imidazole;
2-(3-methoxyphenyl)-5-methyl-4-(1-hydroxy-2-methylpropyl)-1H-imidazole;
2-(2-methoxyphenyl)-5-methyl-4-(1-hydroxy-2-methylpropyl)-1H-imidazole;
2-(2-methylphenyl)-5-ethyl-4-(1-hydroxy-2-methylpropyl)-1H-imidazole;
2-(4-methylphenyl)-5-t-butyl-4-(1-hydroxy-2-methylpropyl)-1H-imidazole hydrochloride;
2-(-4-methylphenyl)-4-(1-hydroxy-2-methylpropyl)-1H-imidazole hydrochloride;
2-(3,4-dimethoxyphenyl)-4-(1-hydroxy-2-methylpropyl)-5-methyl-1H-imidazole; and
2,5-di-(4-methylphenyl)-4-(1-hydroxy-2-methylpropyl)-1H-imidazole,
there is obtained:
2-(3-methylphenyl)-5-methyl-4-(1-chloro-2-methylpropyl)-1H-imidazole hydrochloride;
2-(2-methylphenyl)-5-methyl-4-(1-chloro-2-methylpropyl)-1H-imidazole hydrochloride;
2-(4-t-butylphenyl)-5-methyl-4-(1-chloro-2-methylpropyl)-1H-imidazole hydrochloride;
2-(3-t-butylphenyl)-5-methyl-4-(1-chloro-2-methylpropyl)-1H-imidazole hydrochloride;
2-(2-t-butylphenyl)-5-methyl-4-(1-chloro-2-methylpropyl)-1H-imidazole hydrochloride;
2-(4-chlorophenyl)-5-methyl-4-(1-chloro-2-methylpropyl)-1H-imidazole hydrochloride;
2-(3-chlorophenyl)-5-methyl-4-(1-chloro-2-methylpropyl)-1H-imidazole hydrochloride;
2-(2-chlorophenyl)-5-methyl-4-(1-chloro-2-methylpropyl)-1H-imidazole hydrochloride;
2-(4-methoxyphenyl)-5-methyl-4-(1-chloro-2-methylpropyl)-1H-imidazole hydrochloride;
2-(3-methoxyphenyl)-5-methyl-4-(1-chloro-2-methylpropyl)-1H-imidazole hydrochloride;

2-(2-methoxyphenyl)-5-methyl-4-(1-chloro-2-methylpropyl)-1H-imidazole hydrochloride;
2-(2-methylphenyl)-5-ethyl-4-(1-chloro-2-methylpropyl)-1H-imidazole hydrochloride;
2-(4-methylphenyl)-5-t-butyl-4-(1-chloro-2-methylpropyl)-1H-imidazole hydrochloride;
2-(4-methylphenyl)-4-(1-chloro-2-methylpropyl)-1H-imidazole hydrochloride;
2-(3,4-dimethoxyphenyl)-5-methyl-4-(1-chloro-2-methylpropyl)-1H-imidazole hydrochloride; and
2,5-di-(4-methylphenyl)-4-(1-chloro-2-methylpropyl)-1H-imidazole hydrochloride.

4E. TriHCl Salt of Formula A Where $R^1$ is Phenyl; $R^2$ is Methyl; $R^3$ is Isopropyl; $R^4$ and $R^5$ are Phenyl; m is 2; n is 0; and q is 0

14 Grams (0.05 mol) of diphenylmethyl-4-piperazine and 6 grams (0.15 mol) of sodium hydroxide are dissolved in 180 ml of a mixture of ethanol:water 60:40. The mixture is heated to reflux, then 2-phenyl-5-methyl-4-(1-chloro-2-methylpropyl)-1H-imidazole hydrochloride in 180 milliliters of ethanol:water 60:40 are added dropwise. After 4 to 5 hours under reflux, the reaction mixture is allowed to cool to room temperature. The oil that separated is washed twice with water, then dissolved in ether and hydrochloric acid is added. The precipitate is recrystallized from ethanol to give 1-diphenylmethyl-4-[1-(2-phenyl-5-methyl-1H-imidazol-4-yl)-2-methylpropyl]piperazine trihydrochloride.

4F. TriHCl Salt of Formula A Where $R^3$ is Isopropyl; $R^4$ and $R^5$ are Phenyl; m is 2; n is 0; q is 0; and Varying $R^1$ and $R^2$

Similarly, following the procedure of Part E above, but replacing 2-phenyl-5-methyl-4-(1-chloro-2-methylpropyl)-1H-imidazole hydrochloride with:
2-(3-methylphenyl)-5-methyl-4-(1-chloro-2-methylpropyl)-1H-imidazole hydrochloride;
2-(2-methylphenyl)-5-methyl-4-(1-chloro-2-methylpropyl)-1H-imidazole hydrochloride;
2-(4-t-butylphenyl)-5-methyl-4-(1-chloro-2-methylpropyl)-1H-imidazole hydrochloride;
2-(3-t-butylphenyl)-5-methyl-4-(1-chloro-2-methylpropyl)-1H-imidazole hydrochloride;
2-(2-t-butylphenyl)-5-methyl-4-(1-chloro-2-methylpropyl)-1H-imidazole hydrochloride;
2-(4-chlorophenyl)-5-methyl-4-(1-chloro-2-methylpropyl)-1H-imidazole hydrochloride;
2-(3-chlorophenyl)-5-methyl-4-(1-chloro-2-methylpropyl)-1H-imidazole hydrochloride;
2-(2-chlorophenyl)-5-methyl-4-(1-chloro-2-methylpropyl)-1H-imidazole hydrochloride;
2-(4-methoxyphenyl)-5-methyl-4-(1-chloro-2-methylpropyl)-1H-imidazole hydrochloride;
2-(3-methoxyphenyl)-5-methyl-4-(1-chloro-2-methylpropyl)-1H-imidazole hydrochloride;
2-(2-methoxyphenyl)-5-methyl-4-(1-chloro-2-methylpropyl)-1H-imidazole hydrochloride;
2-(2-methylphenyl)-5-ethyl-4-(1-chloro-2-methylpropyl)-1H-imidazole hydrochloride;
2-(4-methylphenyl)-5-t-butyl-4-(1-chloro-2-methylpropyl)-1H-imidazole hydrochloride;
2-(4-methylphenyl)-4-(1-chloro-2-methylpropyl)-1H-imidazole hydrochloride;
2-(3,4-dimethoxyphenyl)-4-(1-chloro-2-methylpropyl)-5-methyl-1H-imidazole hydrochloride; and
2,5-di-(4-methylphenyl)-4-(1-chloro-2-methylpropyl)-1H-imidazole hydrochloride,

there is obtained:

1-diphenylmethyl-4-[1-(2-(3-methylphenyl)-5-methyl-1H-imidazol-4-yl)-2-methylpropyl]piperazine trihydrochloride;

1-diphenylmethyl-4-[1-(2-(2-methylphenyl)-5-methyl-1H-imidazol-4-yl)-2-methylpropyl]piperazine trihydrochloride;

1-diphenylmethyl-4-[1-(2-(4-t-butylphenyl)-5-methyl-1H-imidazol-4-yl)-2-methylpropyl]piperazine trihydrochloride;

1-diphenylmethyl-4-[1-(2-(3-t-butylphenyl)-5-methyl-1H-imidazol-4-yl)-2-methylpropyl]piperazine trihydrochloride;

1-diphenylmethyl-4-[1-(2-(2-t-butylphenyl)-5-methyl-1H-imidazol-4-yl)-2-methylpropyl]piperazine trihydrochloride;

1-diphenylmethyl-4-[1-(2-(4-chlorophenyl)-5-methyl-1H-imidazol-4-yl)-2-methylpropyl]piperazine trihydrochloride;

1-diphenylmethyl-4-[1-(2-(3-chlorophenyl)-5-methyl-1H-imidazol-4-yl)-2-methylpropyl]piperazine trihydrochloride;

1-diphenylmethyl-4-[1-(2-(2-chlorophenyl)-5-methyl-1H-imidazol-4-yl)-2-methylpropyl]piperazine trihydrochloride;

1-diphenylmethyl-4-[1-(2-(4-methoxyphenyl)-5-methyl-1H-imidazol-4-yl)-2-methylpropyl]piperazine trihydrochloride;

1-diphenylmethyl-4-[1-(2-(3-methoxyphenyl)-5-methyl-1H-imidazol-4-yl)-2-methylpropyl]piperazine trihydrochloride;

1-diphenylmethyl-4-[1-(2-(2-methoxyphenyl)-5-methyl-1H-imidazol-4-yl)-2-methylpropyl]piperazine trihydrochloride;

1-diphenylmethyl-4-[1-(2-(2-methylphenyl)-5-ethyl-1H-imidazol-4-yl)-2-methylpropyl]piperazine trihydrochloride;

1-diphenylmethyl-4-[1-(2-(4-methylphenyl)-5-t-butyl-1H-imidazol-4-yl)-2-methylpropyl]piperazine trihydrochloride;

1-diphenylmethyl-4-[1-(2-(4-methylphenyl)-1H-imidazol-4-yl)-2-methylpropyl]piperazine trihydrochloride;

1-diphenylmethyl-4-[1-(2-(3,4-dimethoxyphenyl)-5-methyl-1H-imidazol-4-yl)-2-methylpropyl]piperazine trihydrochloride; and

1-diphenylmethyl-4-[1-(2,5-di-(4-methylphenyl)-1H-imidazol-4-yl)-2-methylpropyl]piperazine trihydrochloride.

4G. Formula A Where $R^3$ is Isopropyl, Varying q, $R^4$ and $R^5$

Similarly, following the procedure of Part E above, but replacing N-(diphenylmethyl)piperazine with:

N-[di-(2-methylphenyl)methyl]piperazine;

N-[di-(3-methylphenyl)methyl]piperazine;

N-[di-(4-methylphenyl)methyl]piperazine;

N-[di-(2-t-butylphenyl)methyl]piperazine;

N-[di-(3-t-butylphenyl)methyl]piperazine;

N-[di-(4-t-butylphenyl)methyl]piperazine;

N-[di-(2-methoxyphenyl)methyl]piperazine;

N-[di-(3-methoxyphenyl)methyl]piperazine;

N-[di-(4-methoxyphenyl)methyl]piperazine;

N-[di-(2-chlorophenyl)methyl]piperazine;

N-[di-(3-chlorophenyl)methyl]piperazine;

N-[di-(4-chlorophenyl)methyl]piperazine;

N-[di-(4-fluorophenyl)methyl]piperazine;

N-[1-(4-chlorophenyl)-1-(phenyl)methyl]piperazine;

N-(2,2-diphenylethyl)piperazine;

N-[3-(phenyl)-3-(4-methoxyphenyl)propyl]piperazine; and

N-(4,4-diphenylbutyl)piperazine,

there is obtained:

1-[di-(2-methylphenyl)methyl]-4-[1-(2-phenyl-5-methyl-1H-imidazol-4-yl)-2-methylpropyl]piperazine trihydrochloride;

1-[di-(3-methylphenyl)methyl]-4-[1-(2-phenyl-5-methyl-1H-imidazol-4-yl)-2-methylpropyl]piperazine trihydrochloride;

1-[di-(4-methylphenyl)methyl]-4-[1-(2-phenyl-5-methyl-1H-imidazol-4-yl)-2-methylpropyl]piperazine trihydrochloride;

1-[di-(2-t-butylphenyl)methyl]-4-[1-(2-phenyl-5-methyl-1H-imidazol-4-yl)-2-methylpropyl]piperazine trihydrochloride;

1-[di-(3-t-butylphenyl)methyl]-4-[1-(2-phenyl-5-methyl-1H-imidazol-4-yl)-2-methylpropyl]piperazine trihydrochloride;

1-[di-(4-t-butylphenyl)methyl]-4-[1-(2-phenyl-5-methyl-1H-imidazol-4-yl)-2-methylpropyl]piperazine trihydrochloride;

1-[di-(2-methoxyphenyl)methyl]-4-[1-(2-phenyl-5-methyl-1H-imidazol-4-yl)-2-methylpropyl]piperazine trihydrochloride;

1-[di-(3-methoxyphenyl)methyl]-4-[1-(2-phenyl-5-methyl-1H-imidazol-4-yl)-2-methylpropyl]piperazine trihydrochloride;

1-[di-(4-methoxyphenyl)methyl]-4-[1-(2-phenyl-5-methyl-1H-imidazol-4-yl)-2-methylpropyl]piperazine trihydrochloride;

1-[di-(2-chlorophenyl)methyl]-4-[1-(2-phenyl-5-methyl-1H-imidazol-4-yl)-2-methylpropyl]piperazine trihydrochloride;

1-[di-(3-chlorophenyl)methyl]-4-[1-(2-phenyl-5-methyl-1H-imidazol-4-yl)-2-methylpropyl]piperazine trihydrochloride;

1-[di-(4-chlorophenyl)methyl]-4-[1-(2-phenyl-5-methyl-1H-imidazol-4-yl)-2-methylpropyl]piperazine

trihydrochloride;

1-[di-(4-fluorophenyl)methyl]-4-[1-(2-phenyl-5-methyl-1H-imidazol-4-yl)-2-methylpropyl]piperazine trihydrochloride;

1-[1-(4-chlorophenyl)-1-(phenyl)methyl]-4-[1-(2-phenyl-5-methyl-1H-imidazol-4-yl)-2-methylpropyl]-piperazine trihydrochloride;

1-(2,2-diphenylethyl)-4-[1-(2-phenyl-5-methyl-1H-imidazol-4-yl)-2-methylpropyl]piperazine trihydrochloride;

1-[3-(phenyl)-3-(4-methoxyphenyl)propyl]-4-[1-(2-phenyl-5-methyl-1H-imidazol-4-yl)-2-methylpropyl]-piperazine trihydrochloride; and

1-(4,4-diphenylbutyl)-4-[1-(2-phenyl-5-methyl-1H-imidazol-4-yl)-2-methylpropyl]piperazine trihydrochloride.

4H. TriHCl Salt of Formula A Where $R^3$ is Lower Alkyl Other Than Isopropyl

Similarly, by following the procedures of Parts A to G above, and substituting in for 2-chloropropane in Part A the following compounds:

1-chloroethane;

chloromethane;

2-chlorobutane,

there are obtained the corresponding compounds where $R^3$ is, respectively, ethyl, methyl and butyl, such as:

1-diphenylmethyl-4-[1-(2-phenyl-5-methyl-1H-imidazol-4-yl)propyl]piperazine trihydrochloride;

1-diphenylmethyl-4-[1-(2-phenyl-5-methyl-1H-imidazol-4-yl)ethyl]piperazine trihydrochloride;

1-diphenylmethyl-4-[1-(2-phenyl-5-methyl-1H-imidazol-4-yl)-2-methylbutyl]piperazine trihydrochloride;

1-(4,4-diphenylbutyl)-4-[1-(2-phenyl-5-methyl-1H-imidazol-4-yl)-2-methylbutyl]piperazine trihydrochloride;

1-[1-(4-chlorophenyl)-1-(phenyl)methyl]-4-[1-(2-phenyl-5-methyl-1H-imidazol-4-yl)propyl]piperazine trihydrochloride;

1-diphenylmethyl-4-[1-(2-(4-methylphenyl)-5-ethyl-1H-imidazol-4-yl)propyl]piperazine trihydrochloride; and

1-diphenylmethyl-4-[1-(2,5-diphenyl-1H-imidazol-4-yl)-2-methylbutyl]piperazine trihydrochloride.

4I. Formula A Varying m

Similarly, by following the procedures of Parts A-H above, but replacing the piperazines there-used with the corresponding diazepines, the compounds of Formula A wherein m is 3 are obtained.

EXAMPLE 5

1-Diphenylmethyl-4-[2-(4-methylphenyl)-5-methyl-1H-imidazol-4-yl)methyl]piperazine and Derivatives Thereof

A. A solution of 4-methylbenzonitrile in 99% ethanol was saturated with 3 equivalents of dry HCl gas and the reaction mixture was stirred overnight. The precipitate which formed was filtered and the mother liquirs were placed in a freezer at -18° C overnight. A second crop was obtained from the mother liquors. The combined crops were dried at 30° C overnight under vacuum, giving 78% yield of ethyl 4-methylbenzenecarboximidate hydrochloride (compound of Formula 15).

B. The product of step A was added portionwise with stirring to methanol which had been saturated with ammonia in the ratio of 1 liter methanol to 1 kilogram of product of step A. The product of step A dissolved slowly and the solution was stirred overnight. Two thirds of the volume was evaporated under vacuum. The remaining solution was diluted with three times its volume of isopropylacetate. The solution was filtered and dried overnight at 50° C to yield 4-methylbenzenecarboximidamide hydrochloride (compound of Formula 16) in 90% yield.

C. One mole of the product of step B was dissolved in 2 liters of 99% ethanol. 1.1 moles of butanedione were added dropwise, the solution was warmed to reflux and refluxed for 20 hours. The solution was allowed to cool to 30° C and 1 mole of powdered biphenylpiperazine was added portionwise. This was followed by the addition of 1 liter of water, 1.5 equivalents of sodium hydroxide (as 12N solution), and 1.1 equivalents of lithium bromide. The solution was filtered to remove insolubles and then refluxed for 5

hours. The solution was allowed to cool to room temperature and was stirred overnight. The precipitate which formed was filtered and the filter cake was added to an ethanol:water mixture (60:40) in the ratio of 3 liters of solvent mixture to 1 kilogram of filter cake. The solution was warmed to 70 °C for one hour and then allowed to cool to room temperature. The precipitate formed was filtered and dried overnight at 80 °C under vacuum to form 1-diphenylmethyl-4-[2-(-4-methylphenyl)-5-methyl-1H-imidazol-4-yl)methyl]-piperazine in 88% yield.

D. The trihydrochloride salt of the free base of the product of step C was formed by dissolving the free base of step C in 99% ethanol at the ratio of 1.1 liter of ethanol per mole of product. One mole of 1N HCl solution was slowly added. The solution was filtered and then warmed to 60 °C. With slow addition, 250 ml of 12N HCl solution were added. The solution was slowly cooled to -10 °C and the precipitate was filtered off and dried under vacuum at 100 °C for 48 hours to yield 1-diphenylmethyl-4-[2-(-4-methylphenyl)-5-methyl-1H-imidazol-4-yl)methyl]piperazine trichydrochloride, m.p. 204-205 °C.

EXAMPLE 6

Conversion of Free Compound To Salt

Trihydrochloride salt -

The hydrochloride salt was obtained by addition of hydrochloric acid to the free base of a compound of Formula A dissolved in ethanol or ether. See step D of Example 5.

Monohydrochloride salt -

100 g. of 1-diphenylmethyl-4-[2-(-4-methylphenyl)-5-methyl-1H-imidazol-4-yl)methyl]piperazine were dissolved in 300 ml of ethanol at 95 °C. To this solution 1 equivalent of 1N HCl was added with stirring. The reaction mixture was stirred at room temperature for 2 hours then the solvents were evaporated off under reduced pressure. The residue was dissolved in ether and the precipitate was filtered. The precipitate was recrystallized from isopropyl ether:acetone (1:1) to yield 1-diphenylmethyl-4-[2-(-4-methylphenyl)-5-methyl-1H-imidazol-4-yl) methyl]piperazine monohydrochloride, m.p. 186-188 °C with decomposition.

Monomaleate Salt -

5 g. (0.0115 mole) of 1-diphenylmethyl-4-[2-(-4-methylphenyl)-5-methyl-1H-imidazol-4-yl)-methyl]-piperazine were dissolved in 300 ml of ethanol at 95 °C. To this solution 1.34 g (0.0115 mole) of maleic acid was added with stirring. The reaction mixture was stirred at room temperature for 2 hours then the solvents were evaporated off under reduced pressure. The residue was dissolved in ether and the precipitate was filtered. The precipitate was recrystallized from isopropyl ether:acetone (1:1) to yield 1-diphenylmethyl-4-[2-(-4-methylphenyl)-5-methyl-1H-imidazol-4-yl)-methyl]piperazine monomaleate, m.p. 164-166 °C with decomposition.

Trifumarate salt -

To 5 g. of 1-diphenylmethyl-4-[2-(-4-methylphenyl)-5-methyl-1H-imidazol-4-yl)-methyl]piperazine (0.0115 mole) in acetone (100 ml) there was added 1.33 g. (0.0115 mole) of fumaric acid. The solution was heated to reflux, allowed to cool to room temperature, and allowed to stand at room temperature until precipitate formed. The precipitate was collected by filtration and recrystallized from ethanol to give the fumarate salt of 1-diphenylmethyl-4-[2-(-4-methylphenyl)-5-methyl-1H-imidazol-4-yl)methyl]piperazine, m.p. 195 °C in almost quantitative yield.

In a similar manner, all compounds of Formula A in free base form can be converted to the acid addition salts by treatment with the appropriate acid, for example, hydrobromic acid, sulfuric acid, nitric acid, phosphoric acid, acetic acid, propionic acid, glycolic acid, pyruvic acid, oxalic acid, malonic acid, succinic acid, malic acid, maleic acid, tartaric acid, citric acid, lactic acid, benzoic acid, cinnamic acid, mandelic acid, methanesulfonic acid, ethanesulfonic acid, p-toluene- sulfonic acid, and the like.

EXAMPLE 7

Conversion of Salt to Free Base

20 g. of 1-diphenylmethyl-4-[2-(-4-methylphenyl)-5-methyl-1H-imidazol-4-yl)methyl]piperazine trihydrochloride were solubilized in 200 ml of water. Sodium hydroxide (5N) was added dropwise with stirring until pH 8-9. The aqueous medium was extracted twice by 200 ml of dichloromethane. The organic phases were combined then washed with cold water until neutrality. Sodium sulfate was added to dry the organic phase, then the solvent was evaporated under reduced pressure. The resulting residue was crystallized from aqueous ethanol (90%) to give 1-diphenylmethyl-4-[2-(-4-methylphenyl)-5-methyl-1H-imidazol-4-yl)methyl]piperazine.

If desired, the extraction phase can be eliminated to allow direct recovery of the product followed by recrystallization.

EXAMPLE 8

Conversion of one Salt to Another Salt

5 g of 1-diphenylmethyl-4-[2-(-4-methylphenyl)-5-methyl-1H-imidazol-4-yl)methyl]piperazine monofumarate were dissolved in 100 ml of hot ethanol. 3 ml of commercial concentrated HCl solution (10N) were added with stirring. The solution was refluxed for 30 minutes then the solution was allowed to cool to room temperature. The solvent was evaporated off and the residue was crystallized twice from ethanol at 98°C to yield 1-diphenylmethyl-4-[2-(-4-methylphenyl)-5-methyl-1H-imidazol-4-yl)methyl]piperazine trihydrochloride, m.p. 202-204° with decomposition.

EXAMPLES 9-15

The following examples illustrate the preparation of representative pharmaceutical formulations containing an active compound of Formula A, e.g., 1-diphenylmethyl-4-[(2-(4-methylphenyl)-5-methylimidazol-4-yl)-methyl]-piperazine trihydrochloride. Other compounds and salts of Formula A, such as those prepared in accordance with Examples 1-8, can be used as the active compound in the formulations of Examples 9-15.

EXAMPLE 9

| I.V. Formulation | |
|---|---|
| Active compound | 0.14 g |
| Propylene glycol | 20.0 g |
| POLYETHYLENE GLYCOL 400 | 20.0 g |
| TWEEN 80 | 1.0 g |
| 0.9% Saline solution | 100.0 ml |

Other compounds of Formula A and the pharmaceutically acceptable salts thereof may be substituted therein.

EXAMPLE 10

| Ingredients | Quantity per tablet, mgs. |
|---|---|
| Active compound | 25 |
| cornstarch | 20 |
| lactose, spray-dried | 153 |
| magnesium stearate | 2 |

The above ingredients are thoroughly mixed and pressed into single scored tablets.

EXAMPLE 11

| Ingredients | Quantity per capsule, mgs. |
|---|---|
| Active compound<br>lactose, spray-dried<br>magnesium stearate | 100<br>148<br>2 |

The above ingredients are mixed and introduced into a hard-shell gelatin capsule.

EXAMPLE 12

| Ingredients | Quantity per tablet, mgs. |
|---|---|
| Active compound<br>cornstarch<br>lactose<br>magnesium stearate | 1<br>50<br>145<br>5 |

The above ingredients are mixed intimately and pressed into single scored tablets.

EXAMPLE 13

| Ingredients | Quantity per capsule, mgs. |
|---|---|
| Active compound<br>lactose | 150<br>92 |

The above ingredients are mixed and introduced into a hard-shell gelatin capsule.

EXAMPLE 14

An injectable preparation buffered to a pH of 7 is prepared having the following composition:

| Ingredients | |
|---|---|
| Active compound<br>$KH_2PO_4$ buffer (0.4 M solution)<br>KOH (1 N)<br>water (distilled, sterile) | 0.2 g<br>2 ml<br>q.s. to pH 7<br>q.s. to 20 ml |

EXAMPLE 15

An oral suspension is prepared having the following composition:

| Ingredients | |
|---|---|
| Active compound | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |
| colorings | 0.5 mg |
| distilled water | q.s. to 100 ml |

EXAMPLE 16

Ischemia (Stroke, Epilepsy)

A. Five minute model of bilateral common carotid artery occlusion in the gerbil with 72 hour survival

1) Normal animals - Using the procedure of Kirino Brain Res., 239, 57 (1982), microscopic sections (8 mm) of brain tissue were obtained and stained with cresyl fast violet and haematoxylin-eosin. Abnormal brain cells were counted and expressed as a percentage of the total area counted using the procedure of Alps, et al, Br. J. Pharmacol. Proc. Suppl., 88, 250P (1986). The findings for 10 animals with 100 microscopic fields counted were: mean % abnormal neurones = 4.54 ±0.44%.

2) Sham-operated animals - The animals were anesthetized with a halothane-nitrous oxide-oxygen mixture. (Halothane was initially 5% then reduced to 1.5%. The gases were delivered via face mask.) Carotid arteries were surgically exposed (no ischemia) and survival time was 72 hours post-surgery. The microscopic sections were prepared and counted as in 1) above. In this case 7 animals were used and 100 microscopic fields were counted. The results were: mean % abnormal neurones = 4.61 ±0.31%.

3) Untreated ischemic controls - In this case animals were subjected to 5 minute bilateral carotid artery occlusion with 72 hour survival. The microscopic sections were prepared and counted as in 1) above. In this case 12 animals were used and 120 microscopic fields were counted. The results were: mean % abnormal neurones = 78.30 ±2.94%.

4) Parenteral, 15 minute pre-ischemia treated - The animals were given 500 mg/kg i.p. of 1-diphenylmethyl-4-[(2-(4-methylphenyl)-5-methylimidazol-4-yl)methyl]piperazine trihydrochloride 15 minutes prior to ischaemic insult. The treatment was repeated b.i.d. for 72 hours. The microscopic sections were prepared and counted as in 1) above. In this case 10 animals were used and 100 microscopic fields were counted. The results were: mean % abnormal neurones = 26.90 ±3.30%.

5) Parenteral 15 minute pre-ischemic treated - The animals were given 250 mg/kg i.p. of 1-diphenylmethyl-4-[(2-(4-methylphenyl)-5-methylimidazol-4-yl) methyl]piperazine trihydrochloride 15 minutes prior to ischaemic insult. Treatment was repeated b.i.d. for 72 hours. The microscopic sections were prepared and counted as in 1) above. In this case 6 animals were used and 60 microscopic fields were counted. The results were: mean % abnormal neurones = 27.20 ±4.30%.

6) Parenteral 15 minute post-ischemia treated - The animals were given 500 mg/kg i.p. of 1-diphenylmethyl-4-[(2-(4-methylphenyl)-5-methylimidazol-4-yl)methyl]piperazine trihydrochloride 15 minutes after ischaemic insult. The treatment was repeated b.i.d. for 72 hours. The microscopic sections were prepared and counted as in 1) above. In this case 9 animals were used and 90 microscopic fields were counted. The results were: mean % abnormal neurones = 41.70 ±4.60%.

7) Oral, pre-ischemia treated - The animals were given 5 mg/kg p.o. b.i.d. of 1-diphenyl-methyl-4-[(2-(4-methylphenyl)-5-methylimidazol-4-yl)-methyl]piperazine trihydrochloride for 3 days and on the 4th day at 1 hour pre-ischaemic. Treatment was repeated b.i.d. for 72 hours. The microscopic sections were prepared and counted as in 1) above. In this case 11 animals were used and 110 microscopic fields were counted. The results were: mean % abnormal neurones = 3.00 ±1.00%.

8) Oral, pre-ischemia treated - The animals were given 10 mg/kg p.o. of 1-diphenyl-methyl-4-[(2-(4-methylphenyl)-5-methylimidazol-4-yl)-methyl]piperazine trihydrochloride for 3 days and on 4th day at 1 hour pre-ischaemia. Treatment was repeated b.i.d. for 72 hours. The microscopic sections were prepared and counted as in 1) above. In this case 9 animals were used and 90 microscopic fields

were counted. The results were: mean % abnormal neurones = 22.00 ±3.10%.

B. Ten minute Model of four vessel occlusion with 72 hour survival.

The procedure used was that of Alps, et al., Neurology, 37, 809 (1987). The object of this assay was to count abnormally appearing cells in 7 different areas and exymers as a percentage of the total area counted. The number of counts per structure depended upon size, e.g., cortical areas had double the number of other areas. Mean whole brain scores were also determined for percent abnormalities. Normal brains were also used to account for incidence of artifact changes attributed to the fixation process.

1) Normal animals - Under pentobarbital anesthesia samples were obtained, fixed with 10% buffered formal gab and microscopic samples were prepared as described in A above. In this case 6 animals were used with the results shown in the below table.

| Brain Area | Mean % Abnormal Neurones | No. Fields Counted |
|---|---|---|
| Hip $CA_1$ | 1.45 ±0.40% | 60 |
| Hip $CA_{2-5}$ | 0.43 ±0.20% | 60 |
| Hip Cortex | 2.90 ±0.64% | 120 |
| Striatum | 5.80 ±0.54% | 60 |
| Str. Cortex | 3.22 ±0.70% | 120 |
| Thalmus | 4.38 ±0.92% | 60 |
| Purk. cells | 5.61 ±1.88% | 60 |
| Mean Brain score | 3.33 ±0.33% | 540 |
| Mean score per area | 3.39 ±0.76% | 7 |

2) Untreated (saline) ischemic controls - The animals were subjected to 10 minutes of bilateral common carotid artery occlusion (with previously surgically sealed vertebral arteries) with 72 hours survival. The microscopic sections were prepared as in 1) above. In this case 11 animals were used with the results shown in the below table.

| Brain Area | Mean % Abnormal Neurones | No. Fields Counted |
|---|---|---|
| Hip $CA_1$ | 71.60 ±3.00% | 110 |
| Hip $CA_{2-5}$ | 23.80 ±2.70% | 110 |
| Hip. Cortex | 48.50 ±2.20% | 220 |
| Striatum | 45.10 ±2.60% | 110 |
| Str. Cortex | 42.50 ±2.10% | 220 |
| Thalamus | 35.30 ±2.00% | 110 |
| Purk. cells | 32.10 ±3.40% | 110 |
| Mean score per area | 42.70 ±5.80% | 7 |

Parenteral, post-ischaemia treated - The animals were given 100 mg/kg i.a. of 1-diphenyl-methyl-4-[(2-(4-methylphenyl)-5-methylimidazol-4-yl)-methyl]piperazine trihydrochloride 5 minutes post-ischaemia plus 500 mg/kg i.p. of 1-diphenyl-methyl-4-[(2-(4-methylphenyl)-5-methylimidazol-4-yl)-methyl]piperazine trihydrochloride 15 minutes post-ischaemia. The microscopic sections were prepared according to 1) above. In this case 5 animals were used with the results shown in the below table.

| Brain Area | Mean % Abnormal Neurones | No. Fields Counted |
|---|---|---|
| Hip $CA_1$ | 16.76 ±4.00% | 50 |
| Hip $CA_{2-5}$ | 1.78 ±0.99% | 50 |
| Hip. Cortex | 2.41 ±0.30% | 100 |
| Striatum | 1.54 ±0.33% | 50 |
| Str. Cortex | 2.01 ±0.29% | 100 |
| Thalamus | 0.64 ±0.25% | 50 |
| Purk. cells | 2.22 ±0.79% | 50 |

C. The effect of 1-diphenyl-methyl-4-[(2-(4-methylphenyl)-5-methylimidazol-4-yl)-methyl]piperazine

trihydrochloride on pentylenetetrazole-induced seizures and mortality in the mouse.

The procedure used was that described in Allely, and Alps, Br. J. Phramacol. Proc. Suppl., 92, 605P (1987). Groups of 20 or more (see n in table) male CDI mice were predosed with 500 mg/kg i.p. of 1-diphenyl-methyl-4-[(2-(4-methylphenyl)-5-methylimidazol-4-yl)methyl]piperazine trihydrochloride at one of three dosing schedules - A (15 minutes), B (60 minutes), or C (3 days b.i.d. plus 15 minutes on 4th day) - before challenging with 100 mg/kg of pentylenetetrazole s.c. The animals were then observed for a 30 minute period and the occurrence of clonic or tonic seizures or death noted. Statistical analysis was by a Chi squared test of association. Results compared against saline treated animals are shown in the below table.

| Predose Schedule | Predosed mg/kg i.p. | n | % clonic seizures | % tonic seizures | % death |
|---|---|---|---|---|---|
| Saline | | | | | |
| -- | -- | 40 | 92.5 | 80.0 | 70.0 |
| Drug | | | | | |
| A | 500 | 20 | 85.0 | 85.0 | 50.0 |
| B | 500 | 25 | 88.0 | 64.0 | 36.0 |
| C | 500 | 24 | 66.7 | 41.7 | 45.0 |

In an oral dose ranging study in rats of 1-diphenyl-methyl-4-[(2-(4-methylphenyl)-5-methylimidazol-4-yl)-methyl]-piperazine trihydrochloride no death or clinical signs of toxicity were seen at up to 25 mg/kg/day.

EXAMPLE 17

Diuresis

Male normotensive rats weighing 290-380 g were divided into four groups of seven animals. All animals were fasted and deprived of water overnight. The following morning, each group of rats was hydrated with deionized water (20 mg/kg.p.o.) 45 minutes prior to the administration of vehicle (1% polysorbate 80 in deionized water) or 1-diphenyl-methyl-4-[(2-(4-methylphenyl)-5-methylimidazol-4-yl)methyl]-piperazine trihydrochloride at doses of 5, 15, or 30 mg/kg p.o. Fifteen minutes post-drug, the animals were saline loaded (30 ml/kg, p.o. 0.9% sodium chloride) and placed individually in metabolism cages. Urine was collected at 1, 3, and 6 hour intervals post-dose. Urine volumes were measured and sodium and potassium levels were determined by flame photometry. Differences between control and treated values were evaluated by one way analysis of variance. 1-diphenyl-methyl-4-[(2-(4-methylphenyl)-5-methylimidazol-4-yl)-methyl]-piperazine trihydrochloride (30 mg/kg, p.o.) produced significant ($p < .05$) diuresis which was observed at 1 hour and 6 hours post-drug. The compound (30 mg/kg, p.o.) elicited a significant natriuretic effect at the 3 and 6 hour time periods. No significant kaliuretic effects were observed following compound administration.

| Cumulative time | Urine Volume (ml) | | | |
|---|---|---|---|---|
| | control vehicle* | 5 mg/kg p.o. | 15 mg/kg p.o. | 30 mg/kg p.o. |
| 1 hour | 3.3 ±2.7 | 5.7 ±2.5 | 5.7 ±4.4 | 6.5 ±2.3 |
| 3 hours | 7.3 ±3.3 | 9.5 ±3.6 | 9.8 ±3.2 | 10.5 ±2.5 |
| 6 hours | 9.6 ±3.9 | 11.8 ±3.9 | 11.7 ±3.3 | 14.6 ±1.4 |

| Cumulative time | Sodium ion (mEq) | | | |
|---|---|---|---|---|
| | control vehicle* | 5 mg/kg p.o. | 15 mg/kg p.o. | 30 mg/kg p.o. |
| 1 hour | .06 ±.08 | .07 ±.06 | .12 ±.16 | .15 ±.12 |
| 3 hours | .28 ±.31 | .39 ±.25 | .44 ±.20 | .79 ±.38 |
| 6 hours | .55 ±.44 | .73 ±.32 | .68 ±.28 | 1.48 ±.24 |

| Cumulative time | Potassium ion (mEq) | | | |
|---|---|---|---|---|
| | control vehicle* | 5 mg/kg p.o. | 15 mg/kg p.o. | 30 mg/kg p.o. |
| 1 hour | .03 ±.03 | .04 ±.03 | .04 ±.05 | .04 ±.03 |
| 3 hours | .13 ±.09 | .16 ±.09 | .12 ±.06 | .15 ±.08 |
| 6 hours | .22 ±.12 | .26 ±.12 | .20 ±.08 | .27 ±.06 |

EXAMPLE 18

Irritable Bowel Syndrome

The test used is a modification of the method of Macht and Barba-Gose, J. Amer. Pharm. Assoc., 20, 558 (1931), which traces the transit of a charcoal meal through the intestine as an index of transit time. In the present model, intestinal transit in conscious mice (15-20 g) was accelerated with an oral dose of barium chloride (300 mg/kg) administered at the same time as the charcoal meal. The animals were sacrificed 10 minutes later and the distance travelled by the charcoal measured. 1-diphenyl-methyl-4-[(2-(4-methylphenyl)-5-methylimidazol-4-yl)methyl]-piperazine trihydrochloride was given as a 15 minute oral pre-treatment and its effect on non-stimulated or barium-stimulated intestinal transit of the charcoal meal was calculated. The results were expressed as inhibition percentage of the total transit induced by $Ba^{+2}$, and not as the inhibition percentage of the portion representing the $Ba^{+2}$ effect and are shown in the below table.

| Dose 15 min mg/kg p.o. | % Change $Ba^{+2}$ | % Inhibition of $Ba^{+2}$ effect | % Inhibition Normal Transit |
|---|---|---|---|
| 5 | + 93 % | + 14 % | - 19.3 % |
| 25 | + 93 % | - 30.4 % | - 28.7 % |
| 60 | + 93 % | - 31.7 % | - 16.1 % |

**Claims**
**Claims for the following Contracting States : AT, BE, CH, DE, FR, GB, IT, LI, LU, NL, SE**

**1.** A compound having the structure represented by the formula:

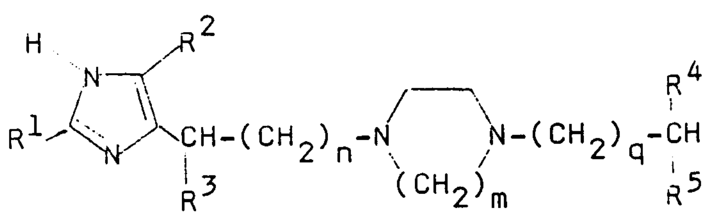

wherein:
$R^1$ is aryl;
$R^2$ is aryl, $C_{1-4}$ alkyl, or hydrogen;

$R^3$ is $C_{1-4}$ alkyl, hydroxy, or hydrogen;
$R^4$ is aryl;
$R^5$ is aryl;
m is two or three;
n is zero, one or two,
provided that when $R^3$ is hydroxy, n is one or two; and
q is zero, one, two, or three;
or a pharmaceutically acceptable salt thereof.

**2.** A compound of Claim 1 wherein m is two, q is zero, n is zero, and $R^2$ is methyl.

**3.** A compound of Claim 2 wherein $R^3$ is hydrogen.

**4.** A compound of Claim 1 wherein $R^3$ is lower alkyl or hydroxy.

**5.** A compound of Claim 3 wherein $R^1$ is 4-methylphenyl.

**6.** A compound of Claim 5 wherein $R^4$ and $R^5$ are 4-chlorophenyl or 4-fluorophenyl.

**7.** A compound of Claim 2 wherein $R^1$ is 3-trifluoromethylphenyl, 4-methoxyphenyl, 4-chlorophenyl, 3,4-dimethoxyphenyl, 3-chlorophenyl, or phenyl.

**8.** A compound of Claim 1 wherein m is 3.

**9.** A compound selected from 1-diphenylmethyl-4-[(2-(4-methylphenyl)-5-methyl-1H-imidazol-4-y1)methyl]-piperazine, 1-diphenylmethyl-4-[2-phenyl-1H-imidazol-4-yl)methyl]piperazine, 1-diphenylmethyl-4-[2-phenyl-5-methyl-1H-imidazol-4-yl)methyl]piperazine, and pharmaceutically acceptable acid addition salts thereof.

**10.** A compound having the structure represented by the formula:

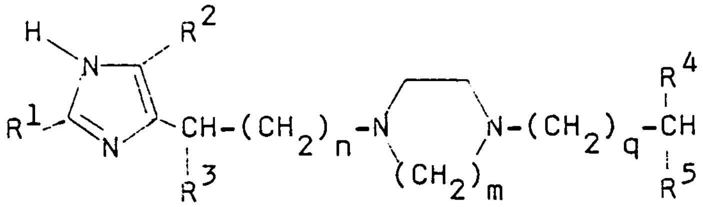

wherein:
$R^1$ is aryl;
$R^2$ is aryl or $C_{1-4}$ alkyl;
$R^3$ is $C_{1-4}$ alkyl, hydroxy, or hydrogen;
$R^4$ is aryl;
$R^5$ is aryl;
m is two or three;
n is zero, one or two,
provided that when $R^3$ is hydroxy, n is one or two; and
q is zero, one, two, or three;
or a pharmaceutically acceptable salt thereof.

**11.** Use of a compound of any one of the preceding claims in the preparation of a medicament for treating stroke, epilepsy or epileptic psychotic symptoms, hypertension, angina, migraine, arrhythmia, thrombosis, embolism, acute cardiac failure, irritable bowel syndrome, neurodegenerative diseases such as Alzheimer's or Huntington's chorea, diuresis, ischemia such as focal and global ischemia or cerebrovascular ischemia induced by cocaine abuse, or spinal injury.

**12.** A compound of any one of claims 1 to 10 for pharmaceutical use.

**13.** A pharmaceutical formulation comprising a compound of any one of claims 1 to 10 and a pharmaceutically acceptable excipient.

**14.** A process for the preparation of a compound of formula A

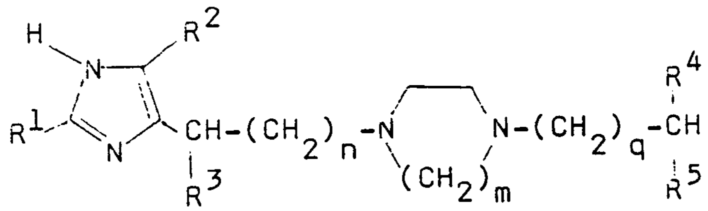

FORMULA A

wherein:

$R^1$ is aryl;
$R^2$ is aryl, lower alkyl, or hydrogen;
$R^3$ is lower alkyl, hydroxy, or hydrogen;
$R^4$ is aryl;
$R^5$ is aryl;
m is two or three;
n is zero, one or two,
provided that when $R^3$ is hydroxy, n is one or two; and
q is zero, one, two, or three;

and the pharmaceutically acceptable salts thereof, which comprises

(a) condensing a compound of the formula

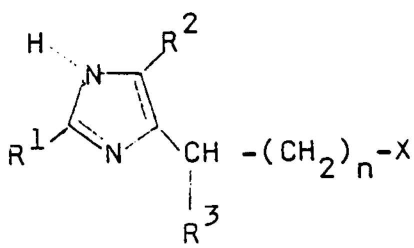

wherein n, $R^1$ and $R^2$ are as defined above, $R^3$ is hydrogen or lower alkyl, and X is a halogen atom, with a compound of the formula

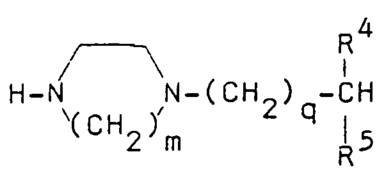

wherein m, q, $R^4$ and $R^5$ are as defined above; or

b) reducing a compound of the formula

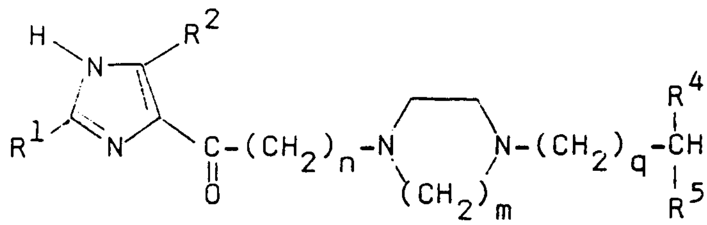

wherein $R^1$, $R^2$, $R^4$, $R^5$, m, and q are as defined above and n is one or two, to form a compound of formula A wherein $R^3$ is hydroxy; or

c) reacting a compound of the formula

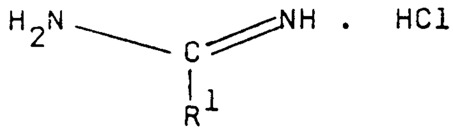

wherein $R^1$ is as defined above, with a compound of the formula

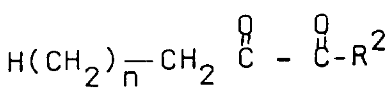

wherein n and $R^2$ are as defined above, and a compound of the formula

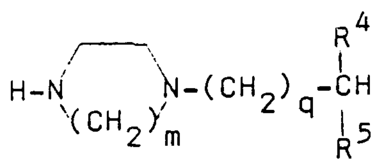

wherein m, q, $R^4$ and $R^5$ are as defined above, to form a compound of Formula A wherein $R^3$ is hydrogen; or

d) converting a free base compound of formula A to its acid addition salt; or

e) converting an acid addition salt of a compound of formula A to a free base compound of formula A; or

f) converting an acid addition salt of a compound of formula A to another acid addition salt of a compound of formula A.

**Claims for the following Contracting States : ES, GR**

1. A process which comprises the preparation of a compound having the structure represented by the formula:

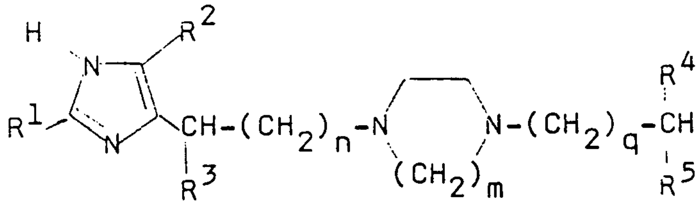

wherein:

$R^1$ is aryl;
$R^2$ is aryl, $C_{1-4}$ alkyl, or hydrogen;
$R^3$ is $C_{1-4}$ alkyl, hydroxy, or hydrogen;
$R^4$ is aryl;
$R^5$ is aryl;
m is two or three;
n is zero, one or two,
provided that when $R^3$ is hydroxy, n is one or two; and
q is zero, one, two, or three;

or a pharmaceutically acceptable salt thereof.

**2.** A process of Claim 1 wherein m is two, q is zero, n is zero, and $R^2$ is methyl.

**3.** A process of Claim 2 wherein $R^3$ is hydrogen.

**4.** A process of Claim 1 wherein $R^3$ is lower alkyl or hydroxy.

**5.** A process of Claim 3 wherein $R^1$ is 4-methylphenyl.

**6.** A process of Claim 5 wherein $R^4$ and $R^5$ are 4-chlorophenyl or 4-fluorophenyl.

**7.** A process of Claim 2 wherein $R^1$ is 3-trifluoromethylphenyl, 4-methoxyphenyl, 4-chlorophenyl, 3,4-dimethoxyphenyl, 3-chlorophenyl, or phenyl.

**8.** A process of Claim 1 wherein m is 3.

**9.** A process which comprises the preparation of a compound selected from 1-diphenylmethyl-4-[(2-(4-methylphenyl)-5-methyl-1H-imidazol-4-yl)methyl]piperazine, 1-diphenylmethyl-4-[2-phenyl-1H-imidazol-4-yl)methyl]piperazine, 1-diphenylmethyl-4-[2-phenyl-5-methyl-1H-imidazol-4-yl)methyl]piperazine, and pharmaceutically acceptable acid addition salts thereof.

**10.** A process which comprises the preparation of a compound having the structure represented by the formula:

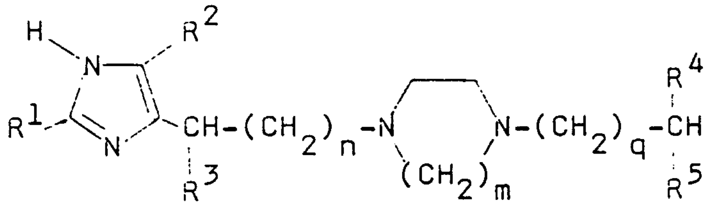

wherein:

$R^1$ is aryl;
$R^2$ is aryl or $C_{1-4}$ alkyl;
$R^3$ is $C_{1-4}$ alkyl, hydroxy, or hydrogen;
$R^4$ is aryl;
$R^5$ is aryl;
m is two or three;
n is zero, one or two,
provided that when $R^3$ is hydroxy, n is one or two; and
q is zero, one, two, or three;

or a pharmaceutically acceptable salt thereof.

**11.** Use of a compound of any one of the preceding claims in the preparation of a medicament for treating stroke, epilepsy or epileptic psychotic symptoms, hypertension, angina, migraine, arrhythmia, thrombosis, embolism, acute cardiac failure, irritable bowel syndrome, neurodegenerative diseases such as Alzheimer's or Huntington's chorea, diuresis, ischemia such as focal and global ischemia or cerebrovascular ischemia induced by cocaine abuse, or spinal injury.

**12.** A process which comprises the preparation of a pharmaceutical formulation comprising a compound of any one of claims 1 to 10 and a pharmaceutically acceptable excipient.

**13.** A process for the preparation of a compound of formula A

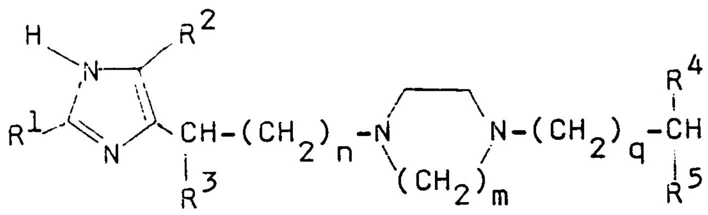

FORMULA A

wherein:

| | |
|---|---|
| $R^1$ | is aryl; |
| $R^2$ | is aryl, lower alkyl, or hydrogen; |
| $R^3$ | is lower alkyl, hydroxy, or hydrogen; |
| $R^4$ | is aryl; |
| $R^5$ | is aryl; |
| m | is two or three; |
| n | is zero, one or two, provided that when $R^3$ is hydroxy, n is one or two; and |
| q | is zero, one, two, or three; |

and the pharmaceutically acceptable salts thereof, which comprises

(a) condensing a compound of the formula

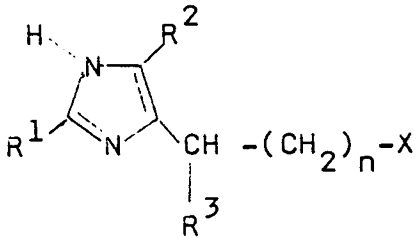

wherein n, $R^1$ and $R^2$ are as defined above, $R^3$ is hydrogen or lower alkyl, and X is a halogen atom, with a compound of the formula

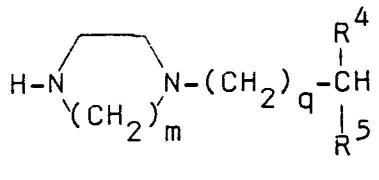

wherein m, q, $R^4$ and $R^5$ are as defined above; or

b) reducing a compound of the formula

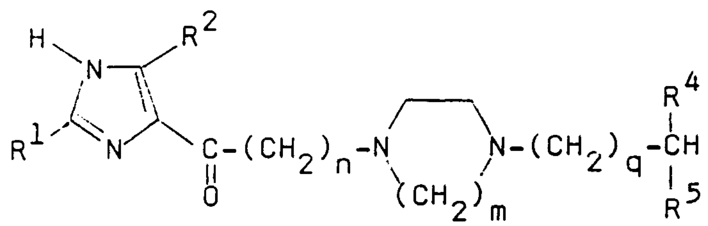

wherein $R^1$, $R^2$, $R^4$, $R^5$, m, and q are as defined above and n is one or two, to form a compound of formula A wherein $R^3$ is hydroxy; or
c) reacting a compound of the formula

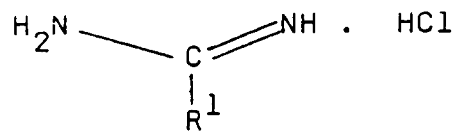

wherein $R^1$ is as defined above, with a compound of the formula

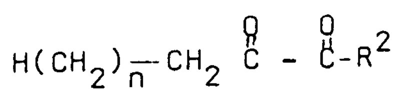

wherein n and $R^2$ are as defined above, and a compound of the formula

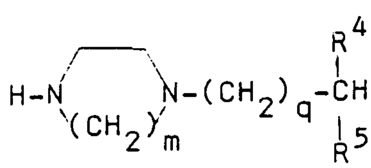

wherein m, q, $R^4$ and $R^5$ are as defined above, to form a compound of Formula A wherein $R^3$ is hydrogen; or
d) converting a free base compound of formula A to its acid addition salt; or
e) converting an acid addition salt of a compound of formula A to a free base compound of formula A; or
f) converting an acid addition salt of a compound of formula A to another acid addition salt of a compound of formula A.

**Revendications**
**Revendications pour les Etats contractants suivants : AT, BE, CH, DE, FR, GB, IT, LI, LU, NL, SE**

**1.** Composé de structure représentée par la formule :

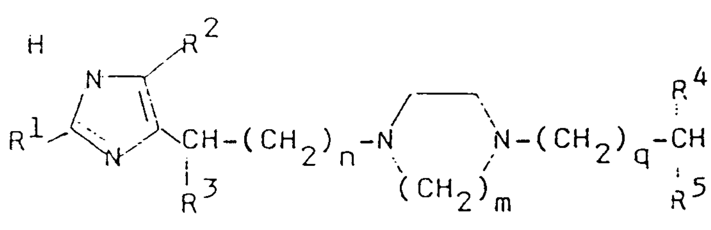

dans laquelle
$R^1$ est un groupe aryle ;
$R^2$ est un groupe aryle, alkyle en $C_1$ à $C_4$ ou l'hydrogène ;
$R^3$ est un groupe alkyle en $C_1$ à $C_4$, hydroxy ou l'hydrogène ;
$R^4$ est un groupe aryle ;
$R^5$ est un groupe aryle ;
m a la valeur deux ou trois ;
n a la valeur zéro, un ou deux,
sous réserve que lorsque $R^3$ est un groupe hydroxy, n ait la valeur un ou deux ; et
q a la valeur zéro, un, deux ou trois ;
ou un sel pharmaceutiquement acceptable de ce composé.

2. Composé suivant la revendication 1, dans lequel m est égal à deux, q est égal à zéro, n est égal à zéro et $R^2$ est le groupe méthyle.

3. Composé suivant la revendication 2, dans lequel $R^3$ est l'hydrogène.

4. Composé suivant la revendication 1, dans lequel $R^3$ est un groupe alkyle inférieur ou hydroxy.

5. Composé suivant la revendication 3, dans lequel $R^1$ est le groupe 4-méthylphényle.

6. Composé suivant la revendication 5, dans lequel $R^4$ et $R^5$ sont le groupe 4-chlorophényle ou 4-fluorophényle.

7. Composé suivant la revendication 2, dans lequel $R^1$ est le groupe 3-trifluorométhylphényle, 4-méthoxyphényle, 4-chlorophényle, 3,4-diméthoxyphényle, 3-chlorophényle ou phényle.

8. Composé suivant la revendication 1, dans lequel m est égal à 3.

9. Composé choisi entre la 1-diphénylméthyl-4-[(2-(4-méthylphényl)-5-méthyl-1H-imidazole-4-yl)méthyl]-pipérazine, la 1-diphénylméthyl-4-[(2-phényl-1H-imidazole-4-yl)méthyl]pipérazine, la 1-diphénylméthyl-4-[(2-phényl-5-méthyl-1H-imidazole-4-yl)méthyl]pipérazine et leurs sels d'addition d'acides pharmaceutiquement acceptables.

10. Composé ayant la structure représentée par la formule :

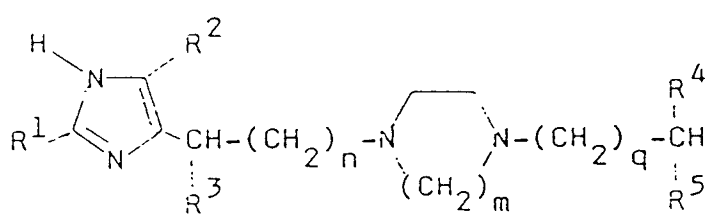

dans laquelle
$R^1$ est un groupe aryle ;
$R^2$ est un groupe aryle ou alkyle en $C_1$ à $C_4$ ;
$R^3$ est un groupe alkyle en $C_1$ à $C_4$, hydroxy ou l'hydrogène ;
$R^4$ est un groupe aryle ;
$R^5$ est un groupe aryle ;
m a la valeur deux ou trois ;
n a la valeur zéro, un ou deux,
sous réserve que lorsque $R^3$ est un groupe hydroxy, n soit égal à un ou deux ; et
q a la valeur zéro, un, deux ou trois ;
ou un sel pharmaceutiquement acceptable de ce composé.

11. Utilisation d'un composé suivant l'une quelconque des revendications précédentes dans la préparation

d'un médicament pour le traitement de l'apoplexie, de l'épilepsie ou des syndromes de psychose épileptiques, l'hypertension, l'angor, la migraine, l'arythmie, la thrombose, l'embolie, la crise cardiaque aiguë, le syndrome d'irritation instestinale, des troubles de dégénérescence nerveuse tels que la chorée d'Alzheimer ou d'Huntington, la diurèse, l'ischémie telle que l'ischémie focale et globale ou l'ischémie cérébrovasculaire induite par l'abus de la cocaïne, ou une lésion de la moelle épinière.

**12.** Composé suivant l'une quelconque des revendications 1 à 10, destiné à un usage pharmaceutique.

**13.** Formulation pharmaceutique, comprenant un composé suivant l'une quelconque des revendications 1 à 10 et un excipient acceptable du point de vue pharmaceutique.

**14.** Procédé de préparation d'un composé de formule A

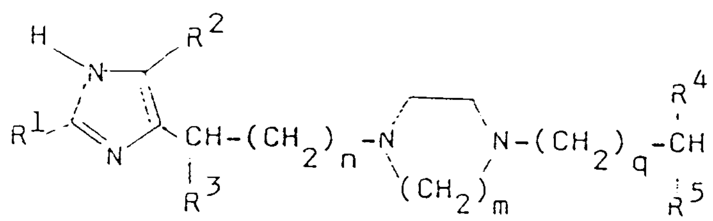

FORMULE A

dans laquelle
$R^1$ est un groupe aryle ;
$R^2$ est un groupe aryle, alkyle inférieur ou l'hydrogène ;
$R^3$ est un groupe alkyle inférieur, hydroxy ou l'hydrogène ;
$R^4$ est un groupe aryle ;
$R^5$ est un groupe aryle ;
m a la valeur deux ou trois ;
n a la valeur zéro, un ou deux,
sous réserve que lorsque $R^3$ est un groupe hydroxy, n soit égal à un ou deux ; et
q a la valeur zéro, un, deux ou trois ;
et de ses sels pharmaceutiquement acceptables, qui consiste
(a) à condenser un composé de formule

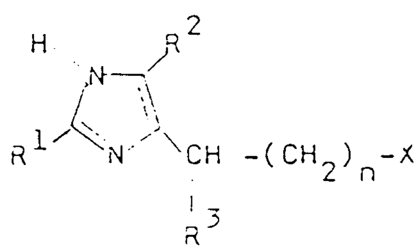

dans laquelle n, $R^1$ et $R^2$ sont tels que définis ci-dessus, $R^3$ est l'hydrogène ou un groupe alkyle inférieur et X est un atome d'halogène,
avec un composé de formule

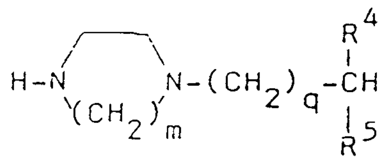

dans laquelle m, q, $R^4$ et $R^5$ sont tels que définis ci-dessus ; ou bien
b) à réduire un composé de formule

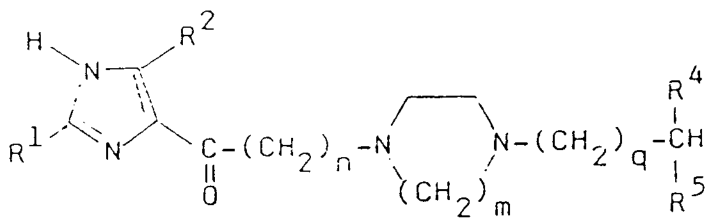

dans laquelle $R^1$, $R^2$, $R^4$, $R^5$, m et q sont tels que définis ci-dessus et n a la valeur un ou deux, pour former un composé de formule A dans laquelle $R^3$ est un groupe hydroxy ; ou bien
c) à faire réagir un composé de formule

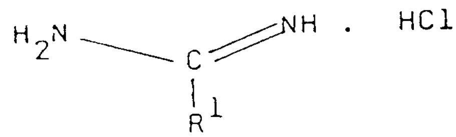

dans laquelle $R^1$ est tel que défini ci-dessus, avec un composé de formule

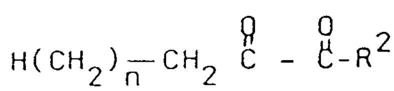

dans laquelle n et $R^2$ sont tels que définis ci-dessus, et un composé de formule

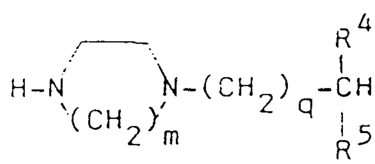

dans laquelle m, q, $R^4$ et $R^5$ sont tels que définis ci-dessus, pour former un composé de formule A dans laquelle $R^3$ est l'hydrogène ; ou bien
d) à convertir un composé sous la forme base libre de formule A en son sel d'addition d'acide ; ou bien
e) à convertir un sel d'addition d'acide d'un composé de formule A en un composé sous la forme base libre de formule A ; ou bien
f) à convertir un sel d'addition d'acide d'un composé de formule A en un autre sel d'addition d'acide d'un composé de formule A.

**Revendications pour les Etats contractants suivants : ES, GR**

**1.** Procédé qui comprend la préparation d'un composé de structure représentée par la formule :

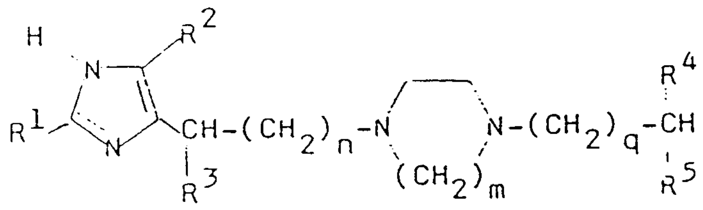

dans laquelle :

R¹ est un groupe aryle ;

$R^2$ est un groupe aryle, alkyle en $C_1$ à $C_4$ ou l'hydrogène ;

$R^3$ est un groupe alkyle en $C_1$ à $C_4$, hydroxy ou l'hydrogène ;

$R^4$ est un groupe aryle ;

$R^5$ est un groupe aryle ;

m a la valeur deux ou trois ;

n a la valeur zéro, un ou deux,

sous réserve que lorsque $R^3$ est un groupe hydroxy, n soit égal à un ou deux ; et

q a la valeur zéro, un, deux ou trois ;

ou d'un sel pharmaceutiquement acceptable de ce composé.

2. Procédé suivant la revendication 1, dans lequel m a la valeur deux, q a la valeur zéro, n a la valeur zéro et $R^2$ est un groupe méthyle.

3. Procédé suivant la revendication 2, dans lequel $R^3$ est l'hydrogène.

4. Procédé suivant la revendication 1, dans lequel $R^3$ est un groupe alkyle inférieur ou hydroxy.

5. Procédé suivant la revendication 3, dans lequel $R^1$ est le groupe 4-méthylphényle.

6. Procédé suivant la revendication 5, dans lequel $R^4$ et $R^5$ sont des groupes 4-chlorophényle ou 4-fluorophényle.

7. Procédé suivant la revendication 2, dans lequel $R^1$ est un groupe 3-trifluorométhylphényle, 4-méthoxyphényle, 4-chlorophényle, 3,4-diméthoxyphényle, 3-chlorophényle ou phényle.

8. Procédé suivant la revendication 1, dans lequel m est égal à 3.

9. Procédé qui comprend la préparation d'un composé choisi entre la 1-diphénylméthyl-4-[(2-(4-méthylphényl)-5-méthyl-1H-imidazole-4-yl)méthyl]pipérazine, la 1-diphénylméthyl-4-[(2-phényl-1H-imidazole-4-yl)méthyl]pipérazine, la 1-diphénylméthyl-4-[(2-phényl-5-méthyl-1H-imidazole-4-yl)méthyl]pipérazine et leurs sels d'addition d'acides pharmaceutiquement acceptables.

10. Procédé qui comprend la préparation d'un composé de structure représentée par la formule :

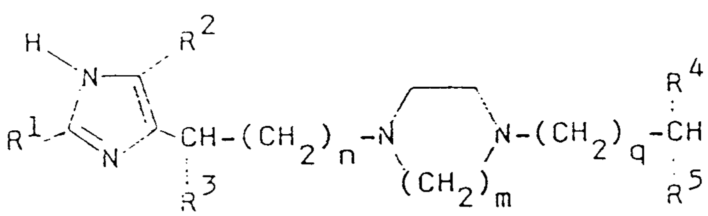

dans laquelle

$R^1$ est un groupe aryle ;

$R^2$ est un groupe aryle ou alkyle en $C_1$ à $C_4$ ;

$R^3$ est un groupe alkyle en $C_1$ à $C_4$, hydroxy ou l'hydrogène ;

$R^4$ est un groupe aryle ;
$R^5$ est un groupe aryle ;
m a la valeur deux ou trois ;
n est égal à zéro, un ou deux,
sous réserve que lorsque $R^3$ est un groupe hydroxy, n soit égal à un ou deux ; et
q a la valeur zéro, un, deux ou trois ;
ou d'un sel pharmaceutiquement acceptable de ce composé.

**11.** Utilisation d'un composé suivant l'une quelconque des revendications précédentes dans la préparation d'un médicament destiné au traitement de l'apoplexie, de l'épilepsie ou des syndromes de psychose épileptiques, l'hypertension, l'angor, la migraine, l'arythmie, la thrombose, l'embolie, la crise cardiaque aiguë, le syndrome d'irritation instestinale, des troubles de dégénérescence nerveuse tels que la chorée d'Alzheimer ou d'Huntington, la diurèse, l'ischémie telle que l'ischémie focale et globale ou l'ischémie cérébrovasculaire induite par l'abus de la cocaïne, ou une lésion de la moelle épinière.

**12.** Procédé qui comprend la préparation d'une formulation pharmaceutique renfermant un composé suivant l'une quelconque des revendications 1 à 10 et un excipient acceptable du point du vue pharmaceutique.

**13.** Procédé de préparation d'un composé de formule A

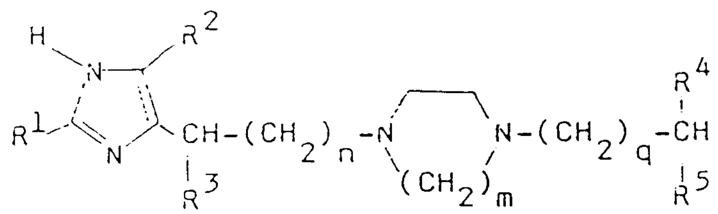

FORMULE A

dans laquelle
$R^1$ est un groupe aryle ;
$R^2$ est un groupe aryle, alkyle inférieur ou l'hydrogène ;
$R^3$ est un groupe alkyle inférieur, hydroxy ou l'hydrogène ;
$R^4$ est un groupe aryle ;
$R^5$ est un groupe aryle ;
m a la valeur deux ou trois ;
n a la valeur zéro, un ou deux,
sous réserve que lorsque $R^3$ est un groupe hydroxy, n soit égal à un ou deux ; et
q a la valeur zéro, un, deux ou trois ;
et de ses sels acceptables du point de vue pharmaceutique, qui consiste
(a) à condenser un composé de formule

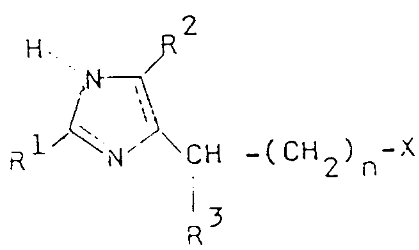

dans laquelle n, $R^1$ et $R^2$ ont la définition indiquée ci-dessus, $R^3$ est l'hydrogène ou un groupe alkyle inférieur et X est un atome d'halogène,

avec un composé de formule

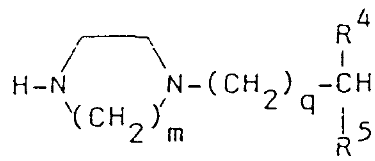

dans laquelle m, q, $R^4$ et $R^5$ sont tels que définis ci-dessus ; ou bien

b) à réduire un composé de formule

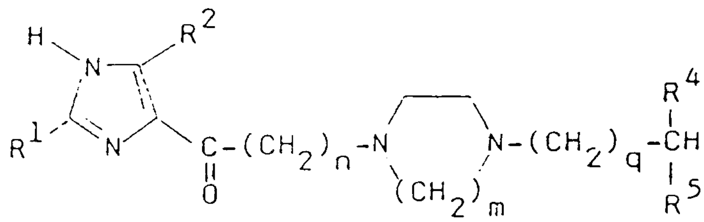

dans laquelle $R^1$, $R^2$, $R^4$, $R^5$, m et q sont tels que définis ci-dessus et n est égal à un ou deux, pour former un composé de formule A dans laquelle $R^3$ est un groupe hydroxy ; ou bien

c) à faire réagir un composé de formule

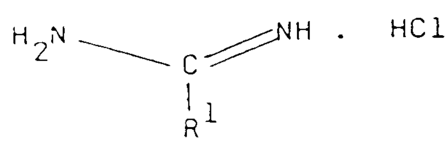

dans laquelle $R^1$ est tel que défini ci-dessus, avec un composé de formule

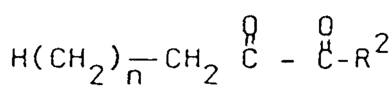

dans laquelle n et $R^2$ sont tels que définis ci-dessus, et un composé de formule

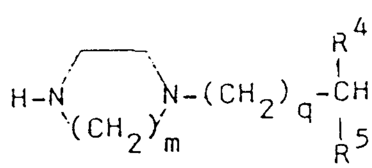

dans laquelle m, q, $R^4$ et $R^5$ sont tels que définis ci-dessus, pour former un composé de formule A dans laquelle $R^3$ est l'hydrogène ; ou bien

d) à convertir un composé sous la forme base libre de formule A en son sel d'addition d'acide ; ou bien

e) à convertir un sel d'addition d'acide d'un composé de formule A en un composé sous la forme base libre de formule A ; ou bien

f) à convertir un sel d'addition d'acide d'un composé de formule A en un autre sel d'addition d'acide d'un composé de formule A.

**Patentansprüche**

**Patentansprüche für folgende Vertragsstaaten : AT, BE, CH, DE, FR, GB, IT, LI, LU, NL, SE**

1. Verbindung mit einer Struktur, die durch die Formel:

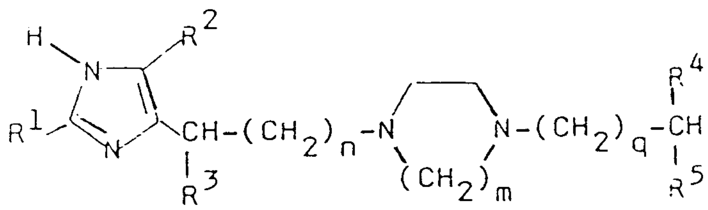

dargestellt wird, worin:
$R^1$ Aryl ist;
$R^2$ Aryl, $C_{1-4}$-Alkyl oder Wasserstoff ist;
$R^3$ $C_{1-4}$-Alkyl, Hydroxy oder Wasserstoff ist;
$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
oder ein pharmazeutisch annehmbares Salz davon.

2. Verbindung nach Anspruch 1, worin m zwei ist, q null ist, n null ist und $R^2$ Methyl bedeutet.

3. Verbindung nach Anspruch 2, worin $R^3$ Wasserstoff bedeutet.

4. Verbindung nach Anspruch 1, worin $R^3$ Niederalkyl oder Hydroxy darstellt.

5. Verbindung nach Anspruch 3, worin $R^1$ 4-Methylphenyl bedeutet.

6. Verbindung nach Anspruch 5, worin $R^4$ und $R^5$ 4-Chlorphenyl oder 4-Fluorphenyl sind.

7. Verbindung nach Anspruch 2, worin $R^1$ 3-Trifluormethylphenyl, 4-Methoxyphenyl, 4-Chlorphenyl, 3,4-Dimethoxyphenyl, 3-Chlorphenyl oder Phenyl darstellt.

8. Verbindung nach Anspruch 1, worin m 3 ist.

9. Verbindung, ausgewählt aus 1-Diphenylmethyl-4-[(2-(4-methylphenyl)-5-methyl-1H-imidazol-4-yl)-methyl]piperazin, 1-Diphenylmethyl-4-[(2-phenyl-1H-imidazol-4-yl)methyl]piperazin, 1-Diphenylmethyl-4-[(2-phenyl-5-methyl-1H-imidazol-4-yl)methyl]piperazin und pharmazeutisch annehmbaren Säureadditionssalzen davon.

10. Verbindung mit der Struktur, die durch die Formel:

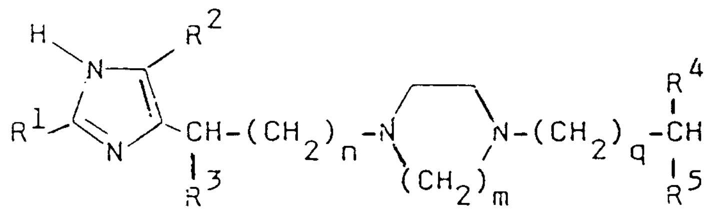

dargestellt wird, worin:
$R^1$ Aryl ist;
$R^2$ Aryl oder $C_{1-4}$-Alkyl ist;

$R^3$ $C_{1-4}$-Alkyl, Hydroxy oder Wasserstoff ist;
$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
oder ein pharmazeutisch annehmbares Salz davon.

**11.** Verwendung einer Verbindung nach irgendeinem der vorangehenden Ansprüche für die Herstellung eines Medikaments zur Behandlung von Schlaganfall, Epilepsie oder epileptisch psychotischen Symptomen, Hochdruck, Angina, Migräne, Arrhythmie, Thrombose, Embolie, akutem Herzversagen,Reizdarm-Syndrom, neurodegenerativen Krankheiten wie z.B. Alzheimer oder Chorea Huntington, Diurese, Ischämie, wie z.B. fokaler und globaler Ischämie oder durch Kokain-Mißbrauch induzierter cerebrovaskulärer Ischämie, oder spinaler Verletzung.

**12.** Verbindung nach irgendeinem der Ansprüche 1 bis 10 zur pharmazeutischen Verwendung.

**13.** Pharmazeutische Formulierung, umfassend eine Verbindung nach irgendeinem der Ansprüche 1 bis 10 und einen pharmazeutisch annehmbaren Exzipienten.

**14.** Verfahren zur Herstellung einer Verbindung der Formel A

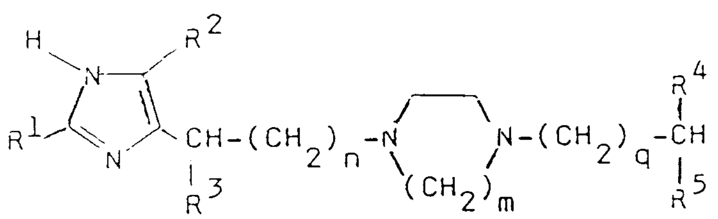

Formel A

worin:
$R^1$ Aryl ist;
$R^2$ Aryl, Niederalkyl oder Wasserstoff ist;
$R^3$ Niederalkyl, Hydroxy oder Wasserstoff ist;
$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
und der pharmazeutisch annehmbaren Salze davon, welches umfaßt
(a) Kondensation einer Verbindung der Formel

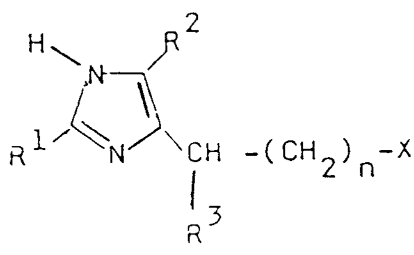

worin n, $R^1$ und $R^2$ wie oben definiert sind, $R^3$ Wasserstoff oder Niederalkyl ist und X ein Halogenatom darstellt,

mit einer Verbindung der Formel

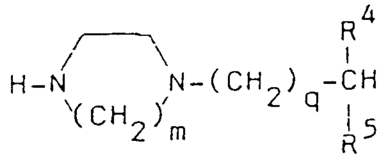

worin m, q, $R^4$ und $R^5$ wie oben definiert sind; oder

(b) Reduktion einer Verbindung der Formel

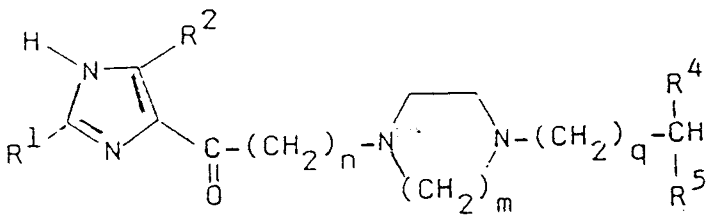

worin $R^1$, $R^2$, $R^4$, $R^5$, m und q wie oben definiert sind und n eins oder zwei ist, um eine Verbindung der Formel A zu bilden, worin $R^3$ Hydroxy ist;

oder

(c) Umsetzung einer Verbindung der Formel

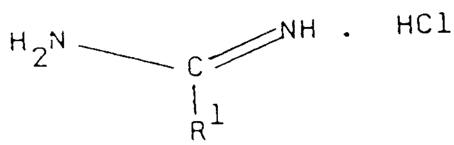

worin $R^1$ wie oben definiert ist, mit einer Verbindung der Formel

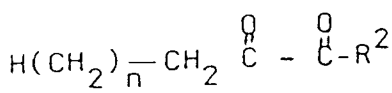

worin n und $R^2$ wie oben definiert sind, und einer Verbindung der Formel

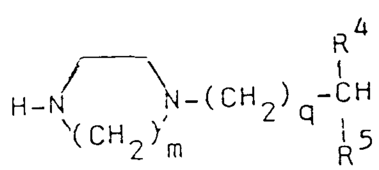

worin m, q, $R^4$ und $R^5$ wie oben definiert sind, um eine Verbindung der Formel A zu bilden, worin $R^3$ Wasserstoff ist; oder

(d) Umwandlung einer freien Base-Verbindung der Formel A in deren Säureadditionssalz; oder

(e) Umwandlung eines Säureadditionssalzes einer Verbindung der Formel A in eine freie Base-Verbindung der Formel A; oder

(f) Umwandlung eines Säureadditionssalzes einer Verbindung der Formel A in ein anderes Säureadditionssalz einer Verbindung der Formel A.

**Patentansprüche für folgende Vertragsstaaten : ES, GR**

1. Verfahren, welches umfaßt die Herstellung einer Verbindung mit der Struktur, die durch die Formel

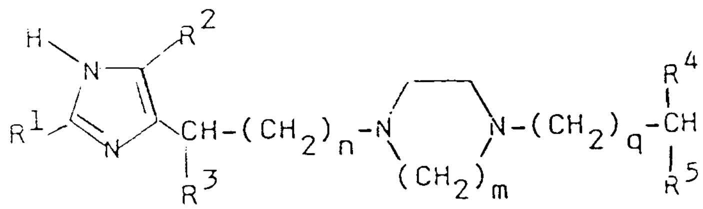

dargestellt wird, worin:
$R^1$ Aryl ist;
$R^2$ Aryl, $C_{1-4}$-Alkyl oder Wasserstoff ist;
$R^3$ $C_{1-4}$-Alkyl, Hydroxy oder Wasserstoff ist;
$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
oder eines pharmazeutisch annehmbaren Salzes davon.

2. Verfahren nach Anspruch 1, worin m zwei ist, q null ist, n null ist und $R^2$ Methyl darstellt.

3. Verfahren nach Anspruch 2, worin $R^3$ Wasserstoff ist.

4. Verfahren nach Anspruch 1, worin $R^3$ Niederalkyl oder Hydroxy ist.

5. Verfahren nach Anspruch 3, worin $R^1$ 4-Methylphenyl ist.

6. Verfahren nach Anspruch 5, worin $R^4$ und $R^5$ 4-Chlorphenyl oder 4-Fluorphenyl darstellen.

7. Verfahren nach Anspruch 2, worin $R^1$ 3-Trifluormethylphenyl, 4-Methoxyphenyl, 4-Chlorphenyl, 3,4-Dimethoxyphenyl, 3-Chlorphenyl oder Phenyl darstellt.

8. Verfahren nach Anspruch 1, worin m 3 ist.

9. Verfahren, welches umfaßt die Herstellung einer Verbindung, die ausgewählt ist aus 1-Diphenylmethyl-4-[(2-(4-methylphenyl)-5-methyl-1H-imidazol-4-yl)methyl]piperazin, 1-Diphenylmethyl-4-[(2-phenyl-1H-imidazol-4-yl)methyl]piperazin, 1-Diphenylmethyl-4-((2-phenyl-5-methyl-1H-imidazol-4-yl)methyl]-piperazin und pharmazeutisch annehmbaren Säureadditionssalzen davon.

10. Verfahren, welches umfaßt die Herstellung einer Verbindung mit der Struktur, die dargestellt wird durch die Formel:

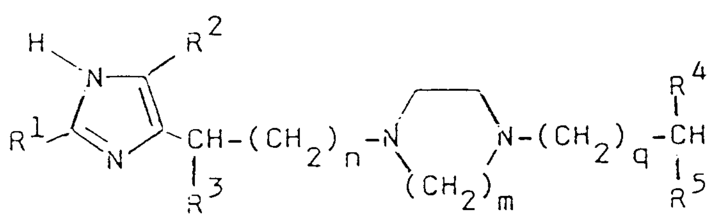

worin:
$R^1$ Aryl ist;
$R^2$ Aryl oder $C_{1-4}$-Alkyl ist;
$R^3$ $C_{1-4}$-Alkyl, Hydroxy oder Wasserstoff ist;

$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
oder eines pharmazeutisch annehmbaren Salzes davon.

**11.** Verwendung einer Verbindung nach irgendeinem der vorangehenden Ansprüche bei der Herstellung eines Medikaments für die Behandlung von Schlaganfall, Epilepsie oder epileptisch psychotischen Symptomen, Hochdruck, Angina, Migräne, Arrhythmie, Thrombose, Embolie, akutem Herzversagen, Reizdarm-Syndrom,neurodegenerativen Erkrankungen wie z.B. Alzheimer oder Chorea Huntington, Diurese, Ischämie, wie z.B. fokaler und globaler Ischämie oder durch Kokain-Mißbrauch induzierter cerebrovaskulärer Ischämie, oder spinaler Verletzung.

**12.** Verfahren, welches umfaßt die Herstellung einer pharmazeutischen Formulierung, die eine Verbindung nach irgendeinem der Ansprüche 1 bis 10 und einen pharmazeutisch annehmbaren Exzipienten umfaßt.

**13.** Verfahren zur Herstellung einer Verbindung der Formel A

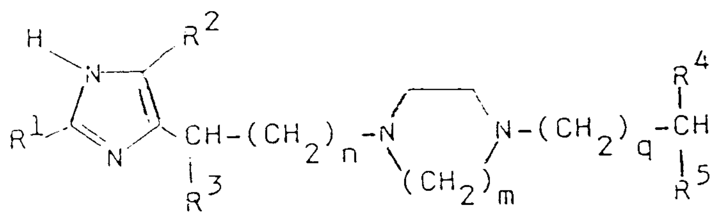

Formel A

worin:
$R^1$ Aryl ist;
$R^2$ Aryl, Niederalkyl oder Wasserstoff ist;
$R^3$ Niederalkyl, Hydroxy oder Wasserstoff ist;
$R^4$ Aryl ist;
$R^5$ Aryl ist;
m zwei oder drei ist;
n null, eins oder zwei ist, mit der Maßgabe, daß wenn $R^3$ Hydroxy ist, n eins oder zwei ist; und
q null, eins, zwei oder drei ist;
und der pharmazeutisch annehmbaren Salze davon, welches umfaßt

(a) Kondensation einer Verbindung der Formel

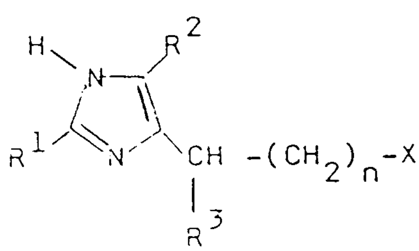

worin n, $R^1$ und $R^2$ wie oben definiert sind, $R^3$ Wasserstoff oder Niederalkyl ist und X ein Halogenatom darstellt,
mit einer Verbindung der Formel

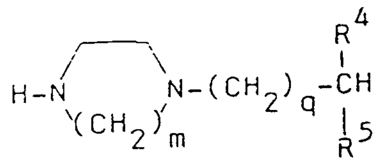

worin m, q, $R^4$ und $R^5$ wie oben definiert sind; oder

(b) Reduktion einer Verbindung der Formel

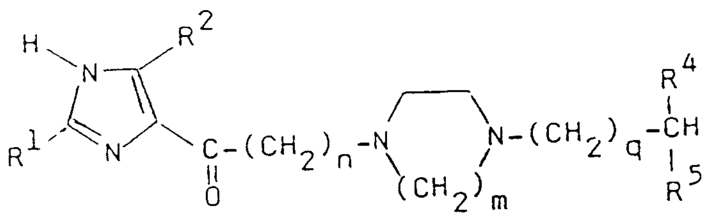

worin $R^1$, $R^2$, $R^4$, $R^5$, m und q wie oben definiert sind und n eins oder zwei ist, um eine Verbindung der Formel A zu bilden, worin $R^3$ Hydroxy ist;

oder

(c) Umsetzung einer Verbindung der Formel

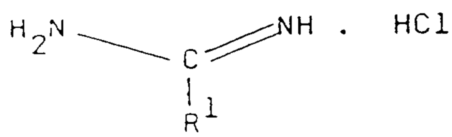

worin $R^1$ wie oben definiert ist, mit einer Verbindung der Formel

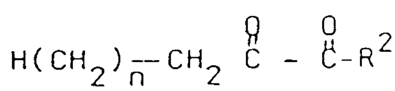

worin n und $R^2$ wie oben definiert sind, und einer Verbindung der Formel

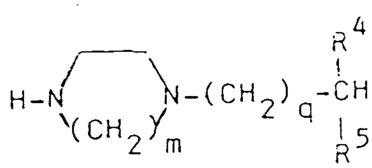

worin m, q, $R^4$ und $R^5$ wie oben definiert sind, um eine Verbindung der Formel A zu bilden, worin $R^3$ Wasserstoff ist; oder

(d) Umwandlung einer freien Base-Verbindung der Formel A in deren Säureadditionssalz; oder

(e) Umwandlung eines Säureadditionssalzes einer Verbindung der Formel A in eine freie Base-Verbindung der Formel A; oder

(f) Umwandlung eines Säureadditionssalzes einer Verbindung der Formel A in ein anderes Säureadditionssalz einer Verbindung der Formel A.